# EUROPEAN PATENT SPECIFICATION

(11) **EP 2 853 894 B1**
(45) Date of publication and mention of the grant of the patent: **30.08.2017**
(21) Application number: 13382376.5
(22) Date of filing: 30.09.2013
(51) Int. Cl.: G01N 33/569, G01N 33/558

(54) **Diagnostic kits and immunoassay methods for diagnosis and differentiation of African Swine Fever Virus (ASFV) and Classical Swine Fever Virus (CSFV)**
Diagnostische Kits und Immunotestverfahren zur Diagnose und Differenzierung von afrikanischem Schweinepestvirus (ASPV) und klassischem Schweinepestvirus (KSPV)
Kits de diagnostic et procédés de dosage immunologique pour le diagnostic et la différenciation du virus de la peste porcine africaine (VPPA) et du virus de la peste porcine classique (CSFV)

(43) Date of publication of application: 01.04.2015
(73) Proprietor: INMUNOLOGIA Y GENETICA APLICADA, S.A., 28037 Madrid (ES)
(72) Inventor: Pérez Escoda, Teresa, E-28037 Madrid (ES); Sanz Fernández, Antonio José, E-28037 Madrid (ES); Rueda Pérez, Paloma, E-28037 Madrid (ES); García Miguet, Julia, E-28037 Madrid (ES); Yang, Xiaoping, CH-8952 Schlieren (CH); Raeber, Alex J., CH-8952 Schlieren (CH); Arias Neira, Marisa, E-28130 Valdeolmos - Madrid (ES); Gallardo Frontaura, Mª Carmen, E-28130 Valdeolmos - Madrid (ES)
(74) Representative: ABG Patentes, S.L.

(56) References cited:
- CN-A- 103 293 306
- LI XUEWU ET AL: "Development of an immunochromatographic strip for rapid detection of antibodies against classical swine fever virus", JOURNAL OF VIROLOGICAL METHODS, vol. 180, no. 1-2, March 2012 (2012-03), pages 32-37, XP055103360, ISSN: 0166-0934
- HAINES FELICITY J ET AL: "Development and Validation of a Multiplex, Real-Time RT PCR Assay for the Simultaneous Detection of Classical and African Swine Fever Viruses", PLOS ONE, vol. 8, no. 7, July 2013 (2013-07), XP8167559,
- ALBAYRAK HARUN ET AL: "A serological survey of selected pathogens in wild boar (Sus scrofa) in northern Turkey", EUROPEAN JOURNAL OF WILDLIFE RESEARCH, vol. 59, no. 6, 18 June 2013 (2013-06-18), pages 893-897, XP8167560,
- YANG S ET AL: "Development of an immunochromatographic strip for the detection of antibodies against foot-and-mouth disease virus serotype O", JOURNAL OF VIROLOGICAL METHODS, ELSEVIER BV, NL, vol. 165, no. 2, 1 May 2010 (2010-05-01), pages 139-144, XP026993867, ISSN: 0166-0934 [retrieved on 2010-01-25]

## Description

### FIELD OF THE INVENTION

The present invention relates to kits and methods for the diagnosis of infection by African swine fever virus (ASFV) or by classical swine fever virus (CSFV) in a subject. The methods of the invention are based on the determination of the presence of anti-ASFV antibodies and/or anti-CSFV antibodies in a blood or serum sample from a subject who is candidate of being infected by said viruses. The kits and methods of the invention also allow simultaneous diagnosis and differentiation between ASFV and CSFV infection.

### BACKGROUND OF THE INVENTION

African swine fever (ASF) is an infectious disease of domestic and wild pigs of all breeds and ages, caused by a virus that produces a range of syndromes. Acute disease is characterized by high fever, haemorrhages in the reticuloendothelial system, and a high mortality rate. Soft ticks of the *Ornithodoros* genus, especially *O*. *moubata* and *O*. *erraticus*, have been shown to be both reservoirs and transmission vectors of the ASF virus (ASFV).

ASFV is the only member of the *Asfarviridae* family, genus *Asfivirus.* ASFV is a complex large icosahedral enveloped DNA virus that exhibits many features common to both iridovirus and poxvirus families. This virus is currently classified as the only member of the *Asfarviridae* family. At least 28 structural proteins have been identified in intracellular virus particles (200 nm). More than a hundred infectious proteins have been identified in infected porcine macrophages, and at least 50 of them react with sera from infected or recovered pigs. The complete analysis of the sequences of several ASFV strains has been accomplished. Different strains of ASFV vary in their ability to cause disease, but at present there is only one recognized serotype of the virus detectable by antibody tests.

The molecular epidemiology of the disease is investigated by sequencing of the C-terminal end of the VP72 gene, which differentiates up to 22 distinct genotypes. Full genome sequence of the p54-gene has been confirmed as a valuable additional genotyping method for molecular epidemiological studies. Enhanced discrimination is obtained by analysis of the central variable region (CVR) within the B602L-gene, described as the most variable locus to distinguish between closely related isolates and identify virus subgroups within several of the 22 genotypes.

ASFV produces a range of syndromes varying from peracute, acute to chronic disease and apparently healthy virus carriers. Pigs are the only domestic animal species that is naturally infected by ASFV. European wild boars and feral pigs are also susceptible to the disease, exhibiting clinical signs and mortality rates similar to those observed in domestic pigs. In contrast, African wild pigs such as warthogs *(Phacochoerus aethiopicus*), bush pigs *(Potamochoerus porcus*) and giant forest hogs (*Hydochoerus meinertzhageni)* are resistant to the disease and show few or no clinical signs. These species of wild pig act as reservoir hosts of ASFV in Africa.

The incubation period in nature is usually 4-19 days. The more virulent strains produce peracute or acute haemorrhagic disease characterized by high fever, loss of appetite, haemorrhages in the skin and internal organs, and death in 4-10 days, sometimes even before the first clinical signs are observed. Mortality rates may be as high as 100%. Less virulent strains produce mild clinical signs (slight fever, reduced appetite and depression) which can be readily confused with many other conditions in pigs and may not lead to suspicion of ASF. Low virulent, non-haemadsorbing strains occasionally produce mainly subclinical non-haemorrhagic infection and seroconversion, but some animals may develop discrete lesions in the lungs or on the skin in areas over bony protrusions and other areas subject to trauma. Animals which have recovered from either acute or chronic infections may become persistently infected, acting as virus carriers. The biological base for the persistence of ASFV is still not well understood. Recovered ASFV carrier pigs and persistently infected wild pigs constitute the biggest problems in controlling the disease. The serological recognition of carrier pigs has been vital for the success of eradication programs.

In countries free from ASFV but suspecting its presence, the laboratory diagnosis must be directed towards isolation of the virus by simultaneously carrying out the inoculation of pig leukocyte or bone marrow cultures, the detection of antigen in smears or cryostat sections of tissues by the fluorescent antibody test (FAT), and by the detection of genomic DNA by the polymerase chain reaction (PCR), which is the most sensitive technique for detecting the presence of the agent in persistently infected animals and is particularly useful if samples submitted are unsuitable for virus isolation and antigen detection because they have undergone putrefaction. ASFV can be detected by PCR from a very early stage of infection in tissues, ethylene diamine tetra-acetic acid (EDTA)-blood and serum samples. Pigs recovered from acute or chronic infections usually exhibit a viraemia for several weeks making the PCR test a very useful tool for the detection of ASFV DNA in pigs infected with low or moderately virulent strains.

As no vaccine is available, the presence of ASFV antibodies is indicative of previous infection and, since antibodies are produced from the first week of infection and persist for long periods, they are a good marker for the diagnosis of the disease. The early appearance (from 7 to 10 days post-infection) and subsequent long-term persistence of antibodies make antibody detection techniques, such as ELISA, immunoblotting or IFA (indirect fluorescent antibody) test, very useful in diagnosing the subacute and chronic forms of disease..

ASFV epidemiology is complex with different epidemiological patterns of infection occurring in Africa and Europe. ASF occurs through transmission cycles involving domestic pigs, wild boars, wild African suids, and soft ticks. In regions where Ornithodorus soft-bodied ticks are present, the detection of ASFV in these reservoirs of infection contributes to a better understanding of the epidemiology of the disease. This is of major importance in establishing effective control and eradication programmes.

ASFV diagnostic techniques include the haemadsorption (HAD) test, fluorescent antibody test (FAT), polymerase chain reaction (PCR). Serological tests include enzyme-linked immunosorbent assays (ELISA), indirect fluorescent antibody test and immunoblotting test.

HAD is based on the fact that pig erythrocytes will adhere to the surface of pig monocyte or macrophage cells infected with ASFV and that most virus isolates produce this phenomenon of haemadsorption. A positive result in the HAD test is definitive for ASFV. A very small number of 'non-haemadsorbing' viruses have been isolated, most of which are avirulent, but some do produce typical acute ASF. The test is carried out by inoculating blood or tissue suspensions from suspect pigs into primary leukocyte cultures, or into alveolar macrophages cell cultures, and also by preparing leukocyte cultures from the blood of pigs inoculated at the laboratory or from the blood of suspect pigs collected in the field diagnosis.

The antigen detection by FAT can be used as an additional method to detect antigen in tissues of suspect pigs in the field or those inoculated at the laboratory. Positive FAT plus clinical signs and appropriate lesions can provide a presumptive diagnosis of ASFV. It can also be used to detect ASFV antigen in leukocyte cultures in which no HAD is observed and can thus identify non-haemadsorbing strains of virus. It also distinguishes between the CPE (cytopathic effect) produced by ASFV and that produced by other viruses, such as Aujeszky's disease virus or a cytotoxic inoculum. However, it is important to note that in subacute and chronic disease, FAT has a significantly decreased sensitivity.

PCR techniques have been developed, using primers from a highly conserved region of the genome, to detect and identify a wide range of isolates belonging to all the known virus genotypes, including both non-haemadsorbing viruses and isolates of low virulence. The PCR techniques are particularly useful for identifying virus DNA in pig tissues that are unsuitable for virus isolation or antigen detection because they have undergone putrefaction, or when there is a good reason to believe that viruses may have been inactivated before samples are received in the laboratory.

Antibodies persist in recovered pigs for long periods after infection, sometimes for life, and a number of tests are available for detecting these antibodies, although only a few of them have been developed for routine use in diagnostic laboratories. The most commonly used is ELISA, which is suitable for examining either serum or fluid from the tissues. Confirmatory testing of ELISA-positive samples should be carried out in critical cases using an alternative test, such as the IFA test, immunoperoxidase staining or immunoblotting. Antibodies are usually not detected in pigs infected with virulent ASFV as they die before they are produced. Antibodies are produced in pigs infected with low or moderately virulent ASF viruses, but these are not fully neutralising antibodies.

Where ASFV is endemic, confirmation of suspected cases of disease is best done using a standard serological test (ELISA), combined with an alternative serological test (IFA) or an antigen-detection test. In some countries, over 95% of positive cases have been identified using a combination of IFA tests and FAT.

The indirect fluorescent antibody test should be used as a confirmatory test for sera from areas that are free from ASFV and are positive in the ELISA, and for sera from endemic areas that give an inconclusive result in the ELISA.

The immunoblotting test should be used as an alternative to the IFA test to confirm equivocal results with individual sera. The immunoblotting test is very specific and enables easier and more objective interpretation of the results and a better recognition of weak-positive samples.

ASF cannot be differentiated from classical swine fever (CSF) by either clinical or post-mortem examination, and both diseases should be considered in the differential diagnosis of any acute febrile haemorrhagic syndrome of pigs. Bacterial septicaemias may also be confused with ASF and CSF. Laboratory tests are essential to distinguish between these diseases.

The clinical symptoms of ASF are very similar to classical swine fever (CSF), ant the two diseases need to be distinguished by laboratory diagnosis, usually performed by ELISA or by isolation of the virus from blood, lymph nodes, spleen or serum of infected pigs.

CSF, also known as hog cholera, is a serious disease of pigs caused by the classical swine fever virus (CSFV) of the family *Flaviviridae*, genus *Pestivirus*. CSF is a highly contagious disease, causing major losses in pig populations almost worldwide, and with the potential of rapid spread. The disease occurs in many regions of Asia, Central and South America and parts of Europe and Africa. Outbreaks can cause heavy losses in pig production and severely hamper international trade with animals and animal products. The socio-economic consequences of CSF outbreaks are severe. Awareness and vigilance are indispensable for early detection of outbreaks and immediate implementation of control measures is essential to prevent further spread. For this goal, reliable and elaborate diagnostic methods are an absolute requirement.

Diagnosis of CSF may be based on clinical signs in the field and gross pathological findings, indirect detection of the virus by serological methods and direct detection of the virus by virus isolation, detection of antigen and nucleic acid.

Clinical and pathological signs of CSF are rather variable and may be mistaken for other pig diseases. Severity of signs mainly depends on the age of the animal and virus virulence.

Serology is routinely used for diagnosis and surveillance and whenever it is suspected that CSFV may be present in a pig population. Antibodies are first detectable 2-3 weeks after infection and persist in surviving animals lifelong. Antibodies are a good indicator that a CSFV infection may have been present in the pig herd. The most commonly used tests for antibody detection are virus neutralization tests (VNT) and ELISA. However, VNT is work-intensive and time consuming, as it relies on cell culture technology. In addition, it cannot be automated, thus it is not suitable for mass analysis of samples. ELISA is widely used to screen for antibodies during and after outbreaks, for monitoring of CSFV infections in wild boar, and to test coverage of immunization of wild boars after vaccination. However, in general, a higher sensitivity of the ELISA is coupled to a lower specificity, as there are cross-reactions with antibodies induced by other *Pestiviruses.* Commercially available antibodies for ELISA were developed as companion tests for E2 glycoprotein-based subunit marker vaccines, but they have limitations in their specificity and/or sensitivity. A competitive ELISA based on a truncated E2 recombinant protein of the Alfort/187 strain of CSFV and a specific monoclonal antibody M1669 has been developed (Clavijo A et al. 2001 J Vet Diagn Invest 13: 357-360). Immunochromatographic strips for the detection of anti-CSFV antibodies have been developed as well (Li X et al. 2012 J Virol Methods 180(1-2): 32-37).

Xuewu et al. (March 2012), vol. 180, p. 32-37 disclose an immunochromatographic strip for detection of antibodies against E2 glycoprotein of classical swine fever virus.

In Chinese patent application CN103293306 (September 2013) an immunochromatographic test strip for detection of antibodies against p54 antigen from African swine fever virus is disclosed.

Due to the economic impact of ASFV and CSFV on swine morbidity and mortality, and the trade implications of the disease, early differential diagnosis of these diseases is of great value. Additionally, preventive measures to adopt for ASF and CSF are quite different. The World Organisation for Animal Health website provides color photographs to assist producers and practitioners in identifying clinical signs and gross lesions associated with CSFV and ASFV. However, ASF and CSF have clinical and pathological similarities, which difficult the differential diagnostic.

Therefore, in view of the state of the art, there is still a need to develop a method for easy, quick and reliable diagnosis of infections caused by ASFV and/or by CSFV. Advantageously, said method should have at least reliability as high as that of the methods described up to date for diagnosing infections caused by ASFV and/or by CSFV, should allow the discrimination (differential diagnosis) between ASFV and CSFV in a simple and easy way, and should be able to be performed directly in the field. Easy, quick and reliable diagnosis and identification of an infection by ASFV or by CSFV is necessary in order to adopt convenient preventive measures in each case.

### SUMMARY OF THE INVENTION

The authors of the present invention have developed an immunochromatographic method suitable for the detection in a sample of a subject of the presence of anti-ASFV antibodies and/or anti-CSFV antibodies.

Therefore, in an aspect, the invention relates to an immunochromatographic strip as in claim 1 comprising
(i) a sample pad (1), wherein said sample pad receives the sample to be analyzed,
(ii) a conjugate pad (2), coupled to or partially overlapping the sample pad (1), wherein said conjugate pad comprises
   - at least one detector reagent specifically binding to an anti-African swine fever virus (ASFV) antibody, wherein said at least one detector reagent comprises an antigen, or a fragment thereof, recognized by the anti-ASFV antibody, and/or
   - at least one detector reagent specifically binding to an anti-classical swine fever virus (CSFV) antibody, wherein said at least one detector reagent comprises an antigen, or a fragment thereof, recognized by the anti-CSFV antibody, and
   - at least one detector reagent specifically binding to a control reagent,
(iii) a signal detection pad (3), coupled to or partially overlapping the conjugate pad (2), wherein said signal detection pad comprises
   - at least one signal detection zone, selected from the group consisting of:
      - an anti-ASFV signal detection zone (6), wherein the presence or the absence of anti-ASFV antibodies in the sample is detected, and wherein said detection zone comprises at least one capture reagent specifically binding to an anti-ASFV antibody, wherein said at least one capture reagent is selected from the group consisting of an antigen, or a fragment thereof, recognized by said anti-ASFV antibody, and/or
      - an anti-CSFV signal detection zone (5), wherein the presence or the absence of anti-CSFV antibodies in the sample is detected, and wherein said detection zone comprises at least one capture reagent specifically binding to an anti-CSFV antibody, wherein said at least one capture reagent is selected from the group consisting of an antigen, or a fragment thereof, recognized by the said anti-CSFV antibody,
      wherein said anti-ASFV signal detection zone and anti-CSFV signal detection zone are different signal detection zones, and
   - a control zone (7), comprising a control capture reagent specifically binding to a control reagent, wherein the presence of a control reagent is detected if the test has been performed correctly, regardless of the presence of anti-ASFV antibodies and/or anti-CSFV antibodies in the sample,
   and,
(iv) an absorbent pad (4), coupled to or partially overlapping the signal detection pad (3)
wherein the capture reagent and/or the detector reagent specifically binding to an anti-ASFV antibody comprise(s) the VP72 protein or a fragment thereof capable of binding to said anti-ASFV antibody, and wherein the capture reagent and/or the detector reagent specifically binding to the anti-CSFV antibody comprise(s) the E2 protein or a fragment thereof capable of binding to said anti-CSFV antibody. In another aspect, the invention relates to a kit comprising at least one immunochromatographic strip as described above.

In another aspect, the invention relates to the use of the immunochromatographic strip provided the invention, or the kit provided the invention, for detecting anti-ASFV antibodies and/or anti-CSFV antibodies in a sample, or for diagnosing an infection caused by ASFV and/or by CSFV, or for discriminating between ASFV and CSFV as the causative agent of an infection in a subject.

In another aspect, the invention relates to an immunoassay method for detecting the presence of anti-ASFV antibodies and/or anti-CSFV antibodies in a subject, wherein said immunoassay method is performed by means of the above mentioned immunochromatographic strip provided by the invention, said method comprising
(i) contacting a sample from said subject with a sample pad of the immunochromatographic strip,
(ii) incubating under suitable conditions for the formation of complexes in the conjugation pad of said immunochromatographic strip between the anti-ASFV antibody and a detector reagent specifically binding to said anti-ASFV antibody and/or complexes between the anti-CSFV and a detector reagent specifically binding to said anti-CSFV antibody, and the migration of said complexes to the signal detection pad of the immunochoromatographic strip, and
(iii) detecting in the signal detection zone of said immunochromatographic strip the formation of complexes between the anti-ASFV antibody-detector reagent and a capture reagent specifically binding to said anti-ASFV antibody and/or the complexes between the anti-CSFV-detector reagent and a capture reagent specifically binding to said anti-CSFV antibody,
wherein the detection of said complexes between the anti-ASFV antibody and a capture reagent specifically binding to said anti-ASFV antibody is indicative of the presence of anti-ASFV antibodies in said subject, and/or the detection of said complexes between the anti-CSFV antibody and a capture reagent specifically binding to said anti-CSFV antibody is indicative of the presence of anti-CSFV antibodies in said subject.

In another aspect, the invention relates to an immunoassay method for discriminating between the causative agent of an infection in a subject, wherein said causative agent is selected from the group consisting of ASFV, CSFV and combinations thereof, wherein said immunoassay method is performed by means of the above mentioned immunochromatographic strip provided by the invention, said method comprising
(i) contacting a sample from said subject with a sample pad of the immunochromatographic strip,
(ii) incubating under suitable conditions for the formation of complexes in the conjugation pad of said immunochromatographic strip between the anti-ASFV antibody and a detector reagent specifically binding to said anti-ASFV antibody and/or complexes between the anti-CSFV and a detector reagent specifically binding to said anti-CSFV antibody, and the migration of said complexes to the signal detection pad of the immunochoromatographic strip, and
(iii) detecting in the signal detection zone of said immunochromatographic strip the formation of complexes between the anti-ASFV antibody-detector reagent and a capture reagent specifically binding to said anti-ASFV antibody and/or the complexes between the anti-CSFV-detector reagent and a capture reagent specifically binding to said anti-CSFV antibody,
wherein the detection of said complexes between the anti-ASFV antibody and a capture reagent specifically binding to said anti-ASFV antibody is indicative of the presence of anti-ASFV antibodies in said subject, and the presence of said anti-ASFV antibodies is indicative that the causative agent of said infection is ASFV; and/or
wherein the detection of said complexes between the anti-CSFV antibody and a capture reagent specifically binding to said anti-CSFV antibody is indicative of the presence of anti-CSFV antibodies in said subject, and the presence of said anti-CSFV antibodies is indicative that the causative agent of said infection is CSFV.

### BRIEF DESCRIPTION OF THE FIGURE

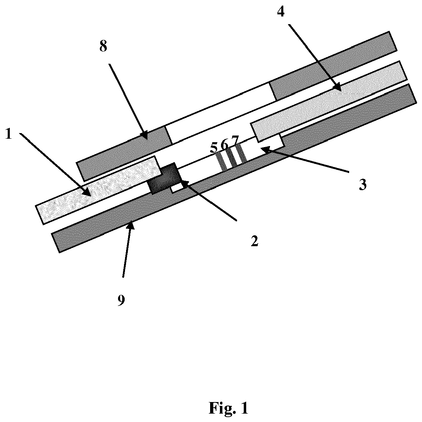

**Figure 1** shows a scheme of an immunochromatographic strip according to the invention. The strip comprises a sample pad (1), a conjugate pad (2), a signal detection pad (3) and an absorbent pad (4). The signal detection pad comprises an anti-CSFV signal detection zone (5), an anti-ASFV signal detection zone (6), and a control zone (7). In a particular embodiment, the signal detection pad is a nitrocellulose membrane. Additional, optional elements of the strip include a protector adhesive (8) and a backing (9), such as a backing plastic.

### DETAILED DESCRIPTION OF THE INVENTION

The authors of the present invention have developed an immunochromatography-based test able to detect the presence in a sample from a subject of specific anti-ASFV and/or anti-CSFV antibodies.

### 1. Definitions

The term "analyte", as used herein, relates to a compound whose presence or absence in a sample from a subject is to be determined by means of the immunochromatographic strip of the invention. In the context of the invention, the analyte is an antibody, in particular an antibody selected from the group consisting of anti-ASFV antibody and anti-CSFV antibody.

The term "absorbent pad", as used herein in the context of immuchromatographic strips, also known as wick or wicking pad, relates to a pad that pulls fluid off of the membrane to allow the capillary flow of the membrane to keep flowing in the proper direction and at the proper rate. Materials suitable for an absorbent pad include cellulose fiber sheets.

The term "antibody" (abbreviated Ab, also known as immunoglobulin, abbreviated Ig), as used herein, relates to immunoglobulin molecules and immunologically active portions of immunoglobulin molecules, i.e., molecules containing an antigen binding site, which specifically bind (immunoreact) with an antigen, such as, for example, a protein. There are 5 isotypes or main classes of immunoglobulins: immunoglobulin M (IgM), immunoglobulin D (IgD), immunoglobulin G (IgG), immunoglobulin A (IgA) and immunoglobulin E (IgE).

The term "antigen", as used herein, includes any molecule or molecular fragment thereof which is recognized,by an antibody; the term includes any molecule or molecular fragment thereof that, when introduced into the body, induces a specific immune response (i.e. humoral or cellular) by the immune system. Antigens have the ability to be bound at the antigen-binding site of an antibody. In the context of the invention, the antigens are related to antigens, or fragments thereof, recognized by an anti-ASFV antibody and that bind to said anti-ASFV antibody, as well as to antigens, or fragments thereof, recognized by an anti-CSFV antibody, and that bind to said anti-CSFV antibody.

The term "ASFV" or "African swine fever virus" relates to a large, double-stranded DNA virus which replicates in the cytoplasm of infected cells, and is the only member of the *Asfarviridae* family. It corresponds to GenBank Taxonomy No. 10497. ASFV is the only virus with a DNA genome transmitted by arthropods, and is the causative agent of African swine fever (ASF). The virus causes a haemorrhagic fever with high mortality rates in pigs, but persistently infects its natural hosts, warthogs, bushpigs and soft ticks of the *Ornithodoros* genus, with no disease signs. The virus genome comprises between 170 and 192 kilobases (kb), with a conserved central region of about 125 kb and variable ends. These variable regions encode five multigene families that are directly involved with the variability of the virus genome. At least 28 structural proteins have been identified in intracellular virus particles (200 nm). More than a hundred infectious proteins have been identified in infected porcine macrophages, and at least 50 of them react with sera from infected or recovered pigs. As used herein, the term ASFV comprises any strain of ASFV, regardless of its origin, for example, Spanish strain España 75 (E75), a highly virulent strain, Spanish strain BA71, corresponding to a strain of ASFV isolated in Badajoz in 1971 and adapted to the monkey kidney-derived stable Vero (V) cell line; said strain (BA71) is apathogenic and is completely sequenced (NCBI accession number U18466, release as of 22 April 1995) (Yanez RJ et al. Analysis of the complete nucleotide sequence of African swine fever virus. Virology. 1995 Apr 1; 208(1):249-78), etc.

The term "buffer solution" or simply "buffer", as used herein, relates to an aqueous solution consisting of a mixture of a weak acid and its conjugate base or a weak base and its conjugate acid. In particular, a physiological buffer emulates physiological conditions, e.g. has a slightly alkaline pH, ranging from 7.4 to 8.0, and an isotonic salt concentration, e.g. between 0.7 and 1.1 % salt (e.g. NaCl), preferably around 0.9% salt (e.g. NaCl). Nonlimiting examples of buffers include Tris-buffered saline, phosphate buffered saline (PBS) and the like.

The term "capture reagent" as used herein in the context of the immunochromatographic strip of the invention, refers to an analyte-specific reagent which can be immobilized on the substrate, particularly on the signal detection pad of the immunochromatographic strip, more particularly in the detection zone of the signal detection pad, and is capable of specific binding to the analyte, or to an analyte-specific reagent, or to a complex of an analyte with an analyte-specific reagent. In a preferred embodiment, the capture reagent is an immobilized capture reagent. In the context of the immunochromatographic strip of the invention, the capture reagent specifically binds to an anti-ASFV antibody, and is selected from the group consisting of an antigen binding to an anti-ASFV antibody, or a fragment of said antigen capable of binding to said anti-ASFV antibody. In a particular embodiment, the capture reagent specifically binding to an anti-ASFV antibody comprises the VP72 protein or a fragment thereof capable of binding to said anti-ASFV antibody. In another non-exclusive embodiment, the capture reagent specifically binds to an anti-CSFV antibody, and is selected from the group consisting of an antigen binding to an anti-CSFV antibody, or a fragment of said antigen capable of binding to said anti-CSFV antibody. In a particular embodiment, the capture reagent specifically binding to an anti-CSFV antibody comprises the E2 protein or a fragment thereof capable of binding to said anti-CSFV antibody. In a particular embodiment, the capture reagent is located on a control zone of the signal detection pad of the strip according to the invention, wherein said capture reagent is capable of specifically binding to a control reagent. In a particular embodiment, the capture reagent is dehydrated or in dry form.

The term "conjugate pad", as used herein in the context of immuchromatographic strips, relates to a pad used to immobilize and release the detector reagent, such as a colloidal detector reagent or a latex detector reagent, once the sample has passed over said pad, uniformly delivering the sample and detector reagent to the signal detection pad (reaction zone). The material comprising the conjugate pad must uniformly wet to accept the conjugate into the matrix of the membrane. In a particular embodiment, the material of the conjugate pad is a porous material. In addition, the conjugate pad should not damage, inactivate, or hinder the release of the conjugate once dried. Conjugate pads are commercially available including, without limitation, conjugation pads from Pall Corporation, Millipore, GE Healthcare, Ahlstrom or Whatman. Materials suitable for a conjugate pad include, without limitation, fiberglass, polyester and rayon.

The term "CSFV" or "classical swine fever virus" relates to the virus causing the classical swine fever (CSF, hog cholera or pig plague), corresponding to GenBank Taxonomy No. 11096. Its genome is located in GenBank under accession No. NC_002657 (12,301 bp, GenBank release as of 8 december 2008). CSF is a serious, economically damaging disease of swine which can spread in an epizootic form as well as establish enzootic infections in domestic and wild pig populations.

The term "detector reagent", as used herein in the context of the immunochromatographic strip of the invention, refers to a reagent used for a visualization of a signal related to the formation of an immunocomplex. In a particular embodiment, the detector reagent is dehydrated or in dry form. According to the invention, the detector reagent comprises an antigen, or a fragment thereof, recognized by an antibody, and a suitable detectable label for detection (including, e.g., colored particles (in the nanometric or micrometric range) - the antigen may be bond to said particles or the antigen may be coating, totally or partially, said particles). In a particular embodiment, the detector reagent specifically binding to an anti-ASFV antibody comprises the VP72 protein or a fragment thereof capable of binding to said anti-ASFV antibody. In a particular embodiment, the detector reagent specifically binding to an anti-CSFV antibody comprises the E2 protein or a fragment thereof capable of binding to said anti-ASFV antibody. In a particular embodiment, suitable detector reagents according to the invention comprise, in addition to an antigen or fragment thereof, a particle, wherein said particle is within the nanometric (from 1 nm to 1000 nm) or the micrometric range (from 1 µm to 1000 µm). In a particular embodiment, the particle is within the nanometric range. These particles are required to be a monodisperse population with a consistent shape. In a particular embodiment, the particle has a substantially spherical shape including, without limitation, a microsphere and a nanosphere. Particles comprised by the detector reagent according to the invention include, without limitation, microspheres and nanospheres such as latex beads, colloidal metal particles such as colloidal gold particles, enzyme conjugates, dye sacs, fluorescent particles, magnetic particles, and the like. These particles are required as well to move through the torturous pore structure of the support (typically a membrane) of the signal detection pad. In a particular embodiment, the antigen or fragment thereof comprised by the detector reagent of the immunochromatographic strip of the invention is bonded to, totally coating or partially coating a particle, preferably selected from the group comprising microspheres and nanospheres, wherein said particles are selected from the group comprising latex particles and colloidal gold particles. In a more particular embodiment, the antigen or fragment thereof of the detector reagent is bonded to, totally coating of partially coating latex particles. In a more particular embodiment, the particles are colored (what in a particular embodiment constitutes the label of the detector reagent since it allows the identification of the immunocomplex formed). An antigen or fragment thereof of the detector reagent specifically binding to an anti-ASFV antibody according to the invention may be bonded to said particles, or alternatively, may be coating, totally or partially, said colored particles; said colored particles may have the same color as, or a color other than, that of the colored particles to which an antigen or fragment thereof of a detector reagent specifically binding to an anti-CSFV antibody is bonded to, or to which it is totally or partially coating them according to the invention. In an embodiment, the particles are dehydrated or in dry form. In a more particular embodiment, the particles are reconstituted in contact with the sample and/or in contact with a buffer solution. In a particular embodiment of the immunochromatographic strip of the invention, the detector reagents are moveable and are embedded in a porous material.

The term "E2 protein", as used herein, relates to a CSFV structural envelope glycoprotein, which is anchored to the envelope by its carboxy end. It is present as an homodimer linked by disulfide bridges on the surface of CSFV virions, as well as dimerized with E1. E2 protein amino acid sequence is located under NCBI Protein database under accession number AEA40488.1 (373 aa, release as of 17 August 2011).

The term "immunoassay", as used herein, includes any immunoassay technique based on the formation or use of immune complexes, that is, resulting from the conjugation of antibodies and antigens, as quantification references of a determined analyte (substance under examination), which can be the antibody or the antigen, using for the measurement a molecule as a marker which produces a detectable signal in response to a specific binding. In a particular embodiment, the analyte the presence of which is to be determined in a sample of a subject is an antibody, more particularly an anti-ASFV or an anti-CSFV antibody.

The skilled person in the art knows that numerous and different markers can be used in an immunoassay; illustrative, non-limitative, examples of said markers include radioactive elements (e.g., sulfur, iodine, etc.), enzymes (e.g., peroxidase, glucosidase, alkaline phosphatase, glucose-6-phosphate dehydrogenase, β-galactosidase, β-glucosidase, β-glucuronidase, etc.), compounds or fluorescent dyes (e.g. fluorescein, rhodamine, etc.), phosphorescent or chemiluminescent (e.g., dioxetanes, acridinium, phenantridinium, ruthenium, luminol, etc.), magnetic or latex particles, colloidal gold, silver or selenium particles, metal chelates, coenzymes, etc., as well as colored particles. Thus, the complex formed can be detected or visualized by any suitable technique, depending on the chosen marker, known to those skilled in the art, using appropriate devices, for example, using radioactive, colorimetric, fluorimetric, (chemo) luminescent, etc. techniques, all known to those skilled in the art. Illustratively, when the marker is an enzyme, detection of complex (antigen-antibody)/marker can be carried out by contacting said complex with a suitable substrate and, optionally, with activators and/or appropriate enzymatic amplification agents. In a particular embodiment, the colored particles to which the antigen is bond to, or to which the antigen is coating, constitute the marker present in the detector reagent.

The term "immunochromatographic strip" or simply "strip", as used herein, relates to an *in vitro* diagnostic test strip based on the principles of immunochromatography, wherein a complex is formed between a detector particle that is free in the sample stream and a capture reagent that is bound to the membrane (at the test line, or signal detection zone, of such membrane comprised by a signal detection pad). Immunochromatographic strips include those strips for assaying the presence/absence of an analyte in a sample, semi-quantitative and quantitative assays. In the context of the present invention, the immunochromatographic strip described herein is for the detection of the presence or the absence of anti-ASFV and/or CSFV antibodies. In an alternative embodiment, the immunochromatographic strip of the invention can be used in a semi-quantitative or quantitative assay, wherein the concentration of the anti-ASFV and/or CSFV antibodies in a sample is determined, by means of a strip reader, in order to quantify the intensity of the test lines, and a standard curve to determine the concentration, as the skilled person acknowledges.

By "lateral flow", as used herein in the context of the immunochromatographic strip of the invention, is meant a fluid flow in a material in which the components that are dissolved in the sample are transported essentially by capillarity with substantially equal speed and a lateral flow intact through the material.

The term "microspheres", also known as microparticles, relates to small spherical particles, with diameters in the micrometer range, typically from 1 µm to 1,000 µm. Commercially available microspheres are from various natural and synthetic materials, such as glass microspheres, polymer microspheres and ceramic microspheres. In a particular embodiment, the detector reagent of the immunochromatographic strip of the invention comprises microspheres. In a particular embodiment, said microspheres are latex microspheres. In a more particular embodiment, the detector reagent of the immunochromatographic strip provided by the invention comprises colored microspheres. Microspheres may be dehydrated or in dry form. Said microspheres are reconstituted in contact with the sample and/or in contact with a buffer solution.

By "moveable", as used herein in the context of the immunochromatographic strip of the invention, is meant that it is not attached to the porous material so that when a detector reagent specifically binding to an anti-ASFV antibody in the conjugate pad and/or when a detector reagent specifically binding to an anti-ASFV antibody in the conjugate pad, if the test sample contains anti-ASFV antibodies and/or anti-CSFV antibodies of interest, a complex is formed between said anti-ASFV antibodies and said detector reagent specifically binding to an anti-ASFV antibody and/or between said anti-CSFV antibodies and said detector reagent specifically binding to an anti-CSFV antibody, wherein said complex(es) can move through the strip up by lateral flow.

The term "nanosphere", as used herein, relates to small spherical particles, with diameters in the nanometer range, typically from 1 nm to 1000 nm. In the context of the invention, the detector reagent of the immunochromatographic strip of the invention comprises nanospheres. In a particular embodiment, the detector reagent of the immunochromatographic strip of the invention comprises nanospheres. In a particular embodiment, said nanospheres are latex nanospheres. In a more particular embodiment, the detector reagent of the immunochromatographic strip of the invention comprises colored nanospheres. In a more particular embodiment, the detector reagent of the immunochromatographic strip of the invention comprises nanospheres, wherein said nanospheres are selected from the group comprising colored latex nanoparticles and colloidal gold nanoparticles. Nanospheres may be dehydrated or in dry form. Said nanospheres are reconstituted in contact with the sample and/or in contact with a buffer solution.

The term "porous material", as used herein, relates to a material capable of allowing the lateral flow through it. Examples of suitable porous materials include, without limitation, acrylonitrile, cotton, fiberglass, cellulose nitrate, blends of nitrocellulose and polyester or cellulose, nylon (polyamides), paper, rayon and similar materials. The expression "strip including a porous material" is meant any element in the shape of an elongated strip containing a porous material. In a particular embodiment of the immunochromatographic strip of the invention, the detector reagents are moveable and are embedded in a porous material.

The term "sample", as used herein in the context of the invention, refers to any sample, preferably a biological sample, which may contain antibodies, particularly to any sample suspected of containing anti-ASFV antibodies and/or anti-CSFV antibodies obtained from the subject under study (unless indicated otherwise). Suitable samples collected from a subject for use in the present invention include any biofluid and, in particular, blood, serum, plasma, lymph, saliva, peripheral blood cells or tissue cells, serum, saliva, semen, sputum, cerebrospinal fluid (CSF), tears, mucus, sweat, milk, or brain extracts. The bodily tissue may comprise thymus, lymph node, spleen, bone marrow, or tonsil tissue. Preferred samples comprise a blood sample, a serum sample, a plasma sample, a meat juice sample, and a saliva sample obtained from said subject. In a particular embodiment, the sample is a blood sample. In a particular embodiment, said blood sample comprises peripheral blood. The term "peripheral blood" relates to the blood volume that circulates distant of the heart, that is, blood flowing through the body of a subject. The blood sample can be obtained by conventional methods known to those skilled in the art. The term "serum" as used herein, refers to the component of the blood resulting after blood coagulation and removal of the resulting clot. Methods for obtaining blood samples from a subject are widely collected in the state of the art as well as methods for obtaining serum from blood samples.

The term "sample pad", as used herein in the context of immuchromatographic strips, relates to a pad where the fluid sample is applied to the lateral flow device. Thus, the sample pad comprises an area or a zone for receiving the sample (also known as reception zone). In a particular embodiment, the sample pad may also comprise a second zone (conjugate zone) comprising at least one capture reagent specifically binding to an anti-ASFV antibody and/or at least one capture reagent specifically binding to an anti-CSFV antibody. In case that the sample pad (comprising the reception zone), and the conjugate pad (comprising the conjugate zone), are different, they must be contacted by a porous material so that the sample can flow by capillarity from the reception zone of the sample pad to the conjugate zone of the conjugate pad. In a particular embodiment, the reception zone is comprised by a sample pad and the conjugate zone is comprised by a conjugate pad, wherein the sample pad and the conjugate pads are different pads. In a particular alternative embodiment, the sample pad comprises both a reception zone and a conjugate zone. In a particular embodiment, the sample is loaded with a drag solution to facilitate the dissolution of the detector reagents in the conjugate zone of the conjugate pad and the lateral flow. In a particular alternative embodiment, the sample pad (comprising the reception zone) and the conjugate pad (comprising the conjugate zone) are different, partially overlapping, pads.

The term "signal detection pad", as used herein in the context of the immunochromatographic strip of the invention, relates to a membrane comprising at least one signal detection zone (also known as test line), wherein said signal detection zone comprises a capture reagent specifically binding to an analyte. In a particular embodiment, the membrane of the signal detection zone comprises two signal detection zones: a first signal detection zone that comprises a first capture reagent for a first analyte, and a second signal detection zone that comprises a second capture reagent for a second analyte. In a preferred embodiment, the signal detection zone comprises as well a control zone (also known as control line), wherein said control zone comprises a capture reagent specifically binding to a control reagent, for migration confirmation along the immunochromatographic strip regardless of the presence of a particular analyte in the sample analyzed by means of said strip. In a particular embodiment, the signal detection pad of the immunochromatographic strip of the invention comprises both a signal detection zone and a control zone. In a more particular embodiment, the signal detection pad of the immunochromatographic strip of the invention comprises both a first signal detection zone, a second signal detection zone and a control zone.

The term "subject", as used herein, relates to any animal having an immune system, preferably mammals, and susceptible of being infected by ASFV or by CSFV, for example, swine (e.g. pigs, etc.).

The term "VP72 protein", as used herein, also known as p72, relates to an ASFV viral capsid protein, accounting for about 32% of the total protein content of the virus particles.VP72 protein amino acid sequence is located under NCBI Protein database under accession number AAM47167.1 (138 aa, release as of 4 June 2002).

### 2. Immunochromatographic strip of the invention

The authors of the present invention have developed an immunochromatography-based test able to detect the presence in a sample from a subject of specific anti-ASFV or anti-CSFV antibodies. The immunochromatographic strip developed by the inventors involves a user-friendly format, very short time to get the test result, long-term stability, and relatively inexpensive; in addition, it allows obtaining the test results in a very short time and testing in the field, with good sensitivity (a parameter measuring the proportion of actual positives which are correctly identified as such, as the ability of said test to identify positive results) and specificity (parameter measuring the proportion of negatives which are correctly identified as such, as the ability of said test to identify negative results) (see Tables 2 and 3 in the Examples section).

Thus, in an aspect, the present invention relates to an immunochromatographic strip, hereinafter referred to as "immunochromatographic strip of the invention", comprising:
(i) a sample pad (1), wherein said sample pad receives the sample to be analyzed,
(ii) a conjugate pad (2), coupled to or partially overlapping the sample pad (1), wherein said conjugate pad comprises
   - at least one detector reagent specifically binding to an anti-African swine fever virus (ASFV) antibody, wherein said at least one detector reagent comprises an antigen, or a fragment thereof, recognized by the anti-ASFV antibody, and/or
   - at least one detector reagent specifically binding to an anti-classical swine fever virus (CSFV) antibody, wherein said at least one detector reagent comprises an antigen, or a fragment thereof, recognized by the anti-CSFV antibody, and
   - at least one detector reagent specifically binding to a control reagent,
(iii) a signal detection pad (3), coupled to or partially overlapping the conjugate pad (2), wherein said signal detection pad comprises
   - at least one signal detection zone, selected from the group consisting of:
      - an anti-ASFV signal detection zone (6), wherein the presence or the absence of anti-ASFV antibodies in the sample is detected, and wherein said detection zone comprises at least one capture reagent specifically binding to an anti-ASFV antibody, wherein said at least one capture reagent is selected from the group consisting of an antigen, or a fragment thereof, recognized by said anti-ASFV antibody, and/or
      - an anti-CSFV signal detection zone (5), wherein the presence or the absence of anti-CSFV antibodies in the sample is detected, and wherein said detection zone comprises at least one capture reagent specifically binding to an anti-CSFV antibody, wherein said at least one capture reagent is selected from the group consisting of an antigen, or a fragment thereof, recognized by the said anti-CSFV antibody, and
   - a control zone (7), comprising a control capture reagent specifically binding to a control reagent, wherein the presence of a control reagent is detected if the test has been performed correctly, regardless of the presence of anti-ASFV antibodies and/or anti-CSFV antibodies in the sample,
   and,
(iv) an absorbent pad (4), coupled to or partially overlapping the signal detection pad (3).

In a particular embodiment, the immunochromatographic strip of the invention allows simultaneous determination of specific anti-ASFV and anti-CSFV antibodies in a sample. Thus in a particular embodiment, the invention relates to an immunochromatographic strip comprising
(i) a sample pad (1), wherein said sample pad receives the sample to be analyzed,
(ii) a conjugate pad (2), coupled to or partially overlapping the sample pad (1), wherein said conjugate pad comprises
   - at least one detector reagent specifically binding to an anti-African swine fever virus (ASFV) antibody, wherein said at least one detector reagent comprises an antigen, or a fragment thereof, recognized by the anti-ASFV antibody,
   - at least one detector reagent specifically binding to an anti-classical swine fever virus (CSFV) antibody, wherein said at least one detector reagent comprises an antigen, or a fragment thereof, recognized by the anti-CSFV antibody, and
   - at least one detector reagent specifically binding to a control reagent,
   wherein said detector reagent are moveable and are embedded in a porous material,
(iii) a signal detection pad (3), coupled to or partially overlapping the conjugate pad (2), wherein said signal detection pad comprises
   - an anti-ASFV signal detection zone (6), wherein the presence or the absence of anti-ASFV antibodies in the sample is detected, and wherein said detection zone comprises at least one capture reagent specifically binding to an anti-ASFV antibody, wherein said at least one capture reagent is selected from the group consisting of an antigen, or a fragment thereof, recognized by said anti-ASFV antibody,
   - an anti-CSFV signal detection zone (5), wherein the presence or the absence of anti-CSFV antibodies in the sample is detected, and wherein said detection zone comprises at least one capture reagent specifically binding to an anti-CSFV antibody, wherein said at least one capture reagent is selected from the group consisting of an antigen, or a fragment thereof, recognized by the said anti-CSFV antibody, and
   - a control zone (7), comprising a control capture reagent specifically binding to a control reagent, wherein the presence of a control reagent is detected if the test has been performed correctly, regardless of the presence of anti-ASFV antibodies and/or anti-CSFV antibodies in the sample,
   and,
(iv) an absorbent pad (4), coupled to or partially overlapping the signal detection pad (3).

The immunochromatographic strip of the invention comprises a number of elements, namely a sample pad (1), a conjugate pad (2), a signal detection pad (3), and an absorbent pad (4). As the skilled person knows, the elements comprising an immunochromatographic strip are arranged in a particular order, so it is possible to conduct the immunochromatography along the strip. In a particular embodiment, the sample pad is located in one end of the immunochromatographic strip, followed by the conjugate pad, followed by the signal detection pad, and followed by the absorbent pad, which is preferably located in the opposite end of the immunochromatographic strip with respect to the sample pad. As the skilled person acknowledges, the pads comprised by the immunochromatographic strip are arranged in a sequential order, wherein a pad is coupled to the following pad or wherein one pad and the following pad are partially overlapping. Alternative arrangements of the elements of the strip may be contemplated, as long as such arrangements allow that the immunochromatography is developed. Although not required, the control zone of the signal detection pad is typically located upstream the detection zone comprised by the signal detection pad.

### 2.1 Sample pad (1)

The immunochromatographic strip of the invention comprises a sample pad (1). This element receives the sample to be analyzed, and controls the even and controlled distribution of said sample onto the conjugate pad. An additional function that can be fulfilled by the sample pad (1) is controlling the rate at which liquid enters the conjugate pad, preventing flooding of the device. When a sample pad (1) is impregnated with components such as proteins (including, without limitation, albumin), detergents (including, without limitation, SDS -sodium dodecyl sulfate- or Tween® 20 (polysorbate 20)), viscosity enhancers, and buffer salts, the sample pad can also be used to increase sample viscosity (modulate flow properties), to enhance the ability of the sample to solubilize the detector reagent, to prevent the conjugate and analyte from binding nonspecifically to any of the downstream materials, and/or to modify the chemical nature of the sample so that it is compatible with immunocomplex formation at the test line.

Differences in pH and ionic strength may shift the specificity and sensitivity of capture and detector reagents and promote varying degrees of non-specific binding of detector reagents due to changes in charge densities. Adding a relatively high concentration of buffer salts to the sample pad (for example, by pre-treating with borate buffer 1.0 M, pH 9.5) can minimize variation by controlling the pH and ionic strength of the solution that emerges from the sample pad.

In a particular embodiment, the sample pad (1) may be pretreated by dipping it into a specific buffer containing a mix of a solution comprised of soluble proteins, surfactants/detergents, and other polymers, which allows a steady flow and prevents nonspecific binding of sample components to the pad.

Suitable materials for a sample pad (1) according to the invention include, without limitation, woven meshes (also known as screens) and cellulose filters.

Woven meshes are normally suitable to distribute the sample volume evenly over the conjugate pad. They also typically have good tensile strength and handle well, even when wet. Meshes have very low bed volumes, meaning that they retain very little sample volume, normally 1-2 µL/cm². On the other hand, it is impractical to treat them with the intention of loading them with enough solutes to modify the protein content, pH, ionic strength or viscosity of the test sample.

Cellulose filters have properties that are nearly the opposite of woven meshes. They are thick (higher than (>) 250 µm), weak, and relatively inexpensive. Cellulose filters also have large bed volumes (higher than (>) 25 µL/cm²). Paper can be very difficult to handle, especially when wet. Cellulosic filters are the most commonly used materials to make the sample pad. Cellulose filters are preferable in this application based on their large bed volume, which permits loading with a wide array of blocking agents, detector reagent release agents, pH and ionic strength modifiers, and viscosity enhancers. When using cellulose filters, care must be taken to ensure sufficient and consistent contact with the underlying conjugate pad material. Failure to achieve good contact and adequate compression can lead to interrupted or inconsistent transfer of fluid into the conjugate pad.

In a particular embodiment, the sample pad (1) of the immunochromatographic strip of the invention is composed of a blend of natural and synthetic fibers including, without limitation, rayon, cellulose and glass fiber. Such blended fibers are commercially available including, without limitation, Cytosep ® membranes (Pall Corporation) for plasma separation from whole blood such as glass fibre A/D (GF A/D), Vivid™ plasma separation membrane, or GF/AVA.

As previously indicated, the sample pad (1) receives the sample to be analyzed by the immunochromatographic strip of the invention, in order to determine the presence or the absence of anti-ASFV antibodies and/or the presence or the absence of anti-CSFV antibodies. Suitable samples for analysis by means of the immunochromatographic strip of the invention are those susceptible of comprising said antibodies, as those selected from the group comprising blood, serum, plasma, meat juices and saliva. In a particular embodiment, the sample for analysis is selected from the group comprising whole blood, serum and plasma. Methods for obtaining a blood sample, a serum sample or a plasma sample are known by the skilled person.

In a particular embodiment, the subject is an animal susceptible of being infected by ASFV or by CSFV. In a more particular embodiment, the subject is a swine.

In a particular embodiment, the sample pad (1) and the conjugate pad (2) are materialized in such a way that both pads constitute an only pad comprising both a region for receiving the sample to be analyzed and a region comprising at least one detector reagent. In an alternative preferred embodiment, the sample pad (1) and the conjugate pad (2) are different pads. In a particular embodiment, the sample pad (1) and the conjugate pad (2) are coupled. In a particular alternative embodiment, as shown in Figure 1, the sample pad (1) partially overlaps the conjugate pad (2).

### 2.2 Conjugate pad (2)

A second element of the immunochromatographic strip according to the invention is the conjugate pad (2).

The conjugate pad (2) of an immunochromatographic strip uniformly transfers the detector reagent and test sample onto the membrane of the signal detection pad (3). When sample flows into the conjugate pad (2), the detector reagent solubilizes, lifts off the pad material, and moves with the sample front into the membrane of the signal detection pad (3). Additional functions of the conjugate pad (2) include delivering the detector reagent onto the membrane in a consistent volume of sample on every immunocromatographic test strip. The sample volume required to release the detector particle into the sample stream determines how much analyte can be measured. Only the analyte contained in the volume of sample that migrates ahead of and with the detector reagent can contribute to the signal. The volume of sample that enters the conjugate pad and membrane after the detector particles have been completely released does not contribute to signal, although it does serve to reduce assay background. An analyte that passes over the capture reagent line after all of the detector reagents have migrated further downstream may bind at the capture reagent line but will lack additional detector reagents to complete the immunocomplex. The sample volume actually analyzed in the test strip equals the amount of sample required to solubilize the detector reagents, not the total amount absorbed by the device.

Characteristics of the conjugate pad material include low non-specific binding (if the detector reagent or the analyte binds to the conjugate pad, it will be unavailable to form the immunocomplex at the test line, thereby reducing signal intensity and sensitivity), consistent flow characteristics (inconsistent flow properties can cause serious performance problems; if the material does not deposit the sample uniformly onto the membrane, the detector reagent may be channeled onto the membrane, appearing as streaks as the sample migrates up the test strip and, consequently, there will be uneven signal development at the test and control lines), consistent bed volume (when loaded into the conjugate pad by dipping, the amount of detector reagent in each test strip depends on the bed volume of the material; if the bed volume varies significantly, variable signal intensities may be observed even though all other components of the strip are constant), low extractables (in addition to chemical extractables, the material should be free of particles that can clog the membrane at the conjugate pad/membrane interface) and good web handling characteristics and consistent compressibility.

Suitable porous materials for the conjugate pad (2) of the immunochromatographic strip of the invention include, without limitation, non-woven filters, which are manufactured by compressing pre-spun fibers of cellulose, glass, or plastic (such as polyester, polypropylene, or polyethylene) into thin mats. They are specified by fiber size, thickness, basis weight, extractables, and air flow rate. In most cases, they cost considerably less than membranes. Materials commonly used to make conjugate pads include glass fiber filters, cellulose filters, and surface-treated (hydrophilic) polyester or polypropylene filters. In a particular embodiment, the conjugate pad (2) of the immunochromatographic strip of the invention is a cellulose fiber conjugate pad. Suitable cellulose fibers are commercially available including, without being limited to, rayon material by Ahlstrom.

In a particular embodiment, the conjugate pad (2) is located following the sample pad (1). In a particular embodiment, the conjugate pad (2) is coupled to the sample pad (1). In a particular preferred embodiment, as shown in Figure 1, the conjugate pad (2) partially overlaps the sample pad (1). In an alternative embodiment, the sample pad (1) and the conjugate pad (2) are materialized in such a way that they constitute a single pad.

### 2.3 Detector reagent of the conjugate pad

Detector reagents are located in the conjugate pad (2) of the immunochromatographic strip of the invention. Said detector reagents are moveable along said strip when the immunochromatography is performed, that is to say, they are not attached to the porous material. When a detector reagent specifically binding to an anti-ASFV antibody in the conjugate pad (2) and/or when a detector reagent specifically binding to an anti-ASFV antibody in the conjugate pad (2), if the test sample contains anti-ASFV antibodies and/or anti-CSFV antibodies, a complex is formed between said anti-ASFV antibodies and said detector reagent specifically binding to an anti-ASFV antibody and/or between said anti-CSFV antibodies and said detector reagent specifically binding to an anti-CSFV antibody, and said complex(es) can move through the strip up by lateral flow.

In an embodiment, the conjugate pad (2) according to the invention comprises at least one detector reagent specifically binding to an anti-ASFV antibody, wherein said at least one detector reagent comprises an antigen recognized by the anti-ASFV antibody, or a fragment thereof recognized by said anti-ASFV antibody.

In another particular embodiment, the conjugate pad according to the invention comprises at least one detector reagent specifically binding to an anti-CSFV antibody, wherein said at least one detector reagent comprises an antigen recognized by the anti-CSFV antibody or a fragment thereof recognized by said anti-CSFV antibody.

In a particular preferred embodiment, the conjugate pad according to the invention comprises:
- at least one detector reagent specifically binding to an anti-ASFV antibody, wherein said at least one detector reagent comprises an antigen, or a fragment thereof, recognized by said anti-ASFV antibody, and
- at least one detector reagent specifically binding to an anti-CSFV antibody, wherein said at least one detector reagent comprises an antigen, or a fragment thereof, recognized by said anti-CSFV antibody.

Antigens recognized by an anti-ASFV antibody according to the invention comprise all those ASFV elements which are recognized by an anti-ASFV antibody, capable of binding specifically to said anti-ASFV antibody, as well as all those ASFV elements capable of eliciting an immune response by the immune system of a subject infected by said ASFV, and include, without limitation, ASFV capsid proteins p72 (VP72, 138 aa, deposited at NCBI Protein database under accession number AAM47167.1 as of 4 June 2002), p30 (encoded by gene E183L, 183aa, NCBI Acc. No. NP_042818.1, release as of 30 November 2009) and p54 (encoded by gene CP204L, 204 aa, NCBI Acc. No. NP_042786.1, release as of 30 November 2009). In a particular embodiment, the detector reagent specifically binding to an anti-ASFV antibody comprises VP72 protein or a fragment thereof recognized by and capable of binding to said anti-ASFV antibody.

Similarly, antigens recognized by an anti-CSFV antibody according to the invention comprise all those CSFV elements capable of binding specifically to an anti-CSFV antibody, as well as all those CSFV elements capable of eliciting an immune response by the immune system of a subject infected by said CSFV including, without limitation, E2 protein. In a particular embodiment, the detector reagent specifically binding to an anti-CSFV antibody comprises E2 protein or a fragment thereof recognized by and capable of binding to said anti-CSFV antibody. Antigenic fragments of E2 protein which are recognized by an anti-CSFV antibody and that bind to said antibody comprise the highly conserved epitope of the glycoprotein E2 as described by Qi *et al.* (Qi Y et al. 2009 Acta Virol 53(4): 241-246). In a particular embodiment, the detector reagent specifically binding to an anti-CSFV antibody comprises an N-terminal fragment of the E2 protein, more particularly an N-terminal region of 136 amino acids as described by Lin *et al.* (Lin M et al. 2005 Clin Diagn Lab Immunol 12(7): 877-881).

Detector reagents according to the invention may be dehydrated or in dry form.

As the skilled person understands, a detector reagent must be capable of producing a signal that is detectable visually or by an instrumental device, that is to say, a detector reagent comprises a probe or label that provides a signal. In a particular embodiment, the detector reagent comprises a particle, wherein said particle provides a detectable signal. Therefore, detector reagents of the invention comprise particles selected from the group comprising gold colloid, carbon or small latex particles. In an alternative particular embodiment, these particles also may include colorimetric or fluorescent chromogens; catalysts; luminescent compounds (e.g., fluorescent, phosphorescent, etc.); radio-active compounds; hollow particles, enzymes or substrates, or organic polymer latex particles; liposomes or other vesicles containing signal producing substances; and the like. In some embodiments, the detection probes comprised by the detector reagent may contain a fluorescent compound that produces a detectable signal. The fluorescent compound may be a fluorescent molecule, polymer, dendrimer, particle, and the like. The probes, such as described above, maybe used alone or in conjunction with a particle (sometimes referred to as "beads" or "microbeads"). If small enough, naturally occurring particles such as nuclei, mycoplasma, plasmids, plastids, mammalian cells (e.g., erythrocyte ghosts), unicellular microorganisms (e.g., bacteria), polysaccharides (e.g., agarose), and the like, may be used. In a particular preferred embodiment, latex particles that are labeled with a fluorescent or colored dye may be utilized.

The probe component of a detector reagent may be modified with certain specific binding members that are adhered thereto to form conjugated probes. Specific binding members generally refer to a member of a specific binding pair, i.e., two different molecules where one of the molecules chemically and/or physically binds to the second molecule. Immunoreactive specific binding members, for example, may include antigens, haptens, aptamers, antibodies (primary or secondary), and complexes thereof, including those formed by recombinant DNA methods or peptide synthesis.

Other common specific binding pairs include but are not limited to, biotin and avidin (or derivatives thereof), biotin and streptavidin, carbohydrates and lectins, complementary nucleotide sequences (including probe and capture nucleic acid sequences used in DNA hybridization assays to detect a target nucleic acid sequence), complementary peptide sequences including those formed by recombinant methods, effector and receptor molecules, hormone and hormone binding protein, enzyme cofactors and enzymes, enzyme inhibitors and enzymes, and the like. Specific binding pairs may include members that are analogs of the original specific binding member; a derivative or fragment of the analyte, i.e., an analyte-analog, may be used so long as it has at least one epitope in common with the analyte.

The specific binding members may generally be attached to the detection probes using any of a variety of well-known techniques. Covalent attachment of the specific binding members to the detection probes (e.g., particles) may be accomplished using carboxylic, amino, aldehyde, bromoacetyl, iodoacetyl, thiol, epoxy and other reactive or linking functional groups, as well as residual free radicals and radical cations, through which a protein coupling reaction may be accomplished. A surface functional group may also be incorporated as a functionalized co-monomer because the surface of the detection probe may contain a relatively high surface concentration of polar groups. In addition, although detection probes are often functionalized after synthesis, in certain cases, such as poly(thiophenol), the particles are capable of direct covalent linking with a protein without the need for further modification. The activation and/or antibody coupling may occur in a buffer, such as phosphate-buffered saline (PBS) (e.g., pH of 7.2) or 2-(N-morpholino) ethane sulfonic acid (MES) (e.g., pH of 5.3). This process forms a conjugated detection probe, where the antibody is covalently attached to the probe.

Attachment techniques other than covalent bonding, such as physical adsorption, may also be utilized in the present invention. In the case of gold colloid, proteins such as antibodies bind well to its surface through a combination of forces, including dative bonding through sulfur-gold interactions, charge attraction, and hydrophobic binding.

In a particular embodiment, the antigen or fragment thereof of the detector reagent specifically binding to the anti-ASFV antibody, the antigen or fragment thereof of the detector reagent specifically binding to the anti-CSFV antibody, and/or the control reagent, is bonded to a particle, or alternatively, they are independently coating, totally or partially, a particle, wherein said particle is comprised by said detector reagent, wherein said particle is selected from the group comprising microespheres and nanospheres. In particular, the particle is selected from a latex particle and a colloidal gold particle, more particularly, from a colored latex particle and a colored colloidal gold particle. More particularly, said particle is selected from colored latex microparticles, colored latex nanoparticles, colloidal gold microparticles and colloidal gold nanoparticles.

In a particular preferred embodiment, both the antigen of the detector reagent specifically binding to an anti-ASFV antibody and the antigen of the detector reagent specifically binding to an anti-ASFV antibody are bonded to and/or coating, partially or totally, microspheres and/or nanospheres, wherein said microspheres and/or nanospheres are colored microspheres and/or colored nanospheres. In an embodiment, the antigen of the detector reagent specifically binding to an anti-ASFV antibody and the antigen of the detector reagent specifically binding to an anti-ASFV antibody are bonded to and/or coating, partially or totally, microspheres and/or nanospheres, wherein the microspheres and/or nanospheres in the detector reagent specifically binding to an anti-ASFV antibody are colored with the same color than the microspheres and/or nanospheres in the detector reagent specifically binding to an anti-CSFV antibody. In a particular preferred embodiment, the antigen of the detector reagent specifically binding to an anti-ASFV antibody and the antigen of the detector reagent specifically binding to an anti-ASFV antibody are bonded to and/or coating, partially or totally, microspheres and/or nanospheres, wherein the microspheres and/or nanospheres in the detector reagent specifically binding to an anti-ASFV antibody are colored with a different color than the microspheres and/or nanospheres in the detector reagent specifically binding to an anti-CSFV antibody.

In a particular embodiment, the antigen specific for an anti-ASFV antibody or the antigen specific for an anti-CSFV antibody of the detector reagent are bound to the microspheres or to the nanospheres by conjugation, wherein the conjugation is performed by methods known by the skilled person.

In a particular embodiment, the immunochromatographic strip of the invention comprises a control reagent, preferably a control protein, wherein said control reagent is located at the sample pad or at the conjugate pad, preferably at the conjugate pad, at least one control detector reagent specifically binding to said control reagent, wherein the detector reagent is located at the conjugate pad, and at least one control capture reagent specifically binding to said control reagent, wherein said control capture reagent is located at the control zone of the signal detection pad of the immunochromatographic strip of the invention. Thus, the control zone of the immunochromatographic strip of the invention, comprising the control capture reagent specifically binding to the control reagent, allows detection of a control reagent such as a control protein, wherein detection of said control protein is indicative of migration confirmation when the lateral flow along the immunochromatographic strip has occurred adequately, regardless of the presence of anti-ASFV antibodies and/or anti-CSFV antibodies in the sample analysed by means of said strip. In a particular alternative embodiment, the immunochromatographic strip of the invention comprises a control reagent located at the conjugate pad, wherein said control reagent comprises colored particles, and a capture reagent, located at the control zone of the signal detection pad, wherein said capture reagent specifically binds to the control reagent.

Control reagents suitable for a strip according to the invention may be determined by the skilled person and comprise, without limitation, biotin, avidin, streptavidin, bovine serum albumin (BSA), human serum albumin (HSA), keyhole limpet hemocyanin (KLH), and ovalbumin (OVA). In a particular embodiment, the control reagent is biotin. In an alternative embodiment, the control reagent is an antibody, wherein said antibody is selected from the group comprising anti-biotin antibody, anti-avidin antibody, anti-streptavidin antibody, anti-BSA antibody, anti-HSA antibody, anti-KLH antibody and anti-OVA antibody.

In a particular embodiment, the control reagent is selected from the group comprising biotin, avidin, streptavidin, BSA, HSA, KLH and OVA, and the capture reagent specifically binding to said control reagent is selected from the group comprising anti-biotin antibody, avidin, streptavidin, anti-BSA antibody, anti-HSA antibody, anti-KLH antibody, and anti-OVA antibody.

Control detector reagent according to the invention comprises any reagent specifically binding to the control reagent. In a particular embodiment, the control detector reagent may be dehydrated or in dry form. In a particular embodiment, the control detector reagent is bonded to and/or coating microspheres and/or nanospheres. In a particular embodiment, said microspheres and/or nanospheres are colored microspheres and/or nanospheres. In a more particular embodiment, said colored microspheres and/or nanospheres of the control detector reagent are colored with the same color than the microspheres and/or nanospheres in the detector reagent specifically binding to an anti-ASFV antibody, and with the same color than the microspheres and/or nanospheres in the detector reagent specifically binding to an anti-CSFV antibody. In an alternative embodiment, the colored microspheres and/or nanospheres of the control detector reagent are colored with a different color from the color of the microspheres and/or nanospheres in the detector reagent specifically binding to an anti-ASFV antibody. In another alternative embodiment, the colored microspheres and/or nanospheres of the control detector reagent are colored with a different color from the color of the microspheres and/or nanospheres in the detector reagent specifically binding to an anti-CSFV antibody. In an alternative preferred embodiment, the colored microspheres and/or nanospheres of the control detector reagent are colored with a different color from the color of the microspheres and/or nanospheres in the detector reagent specifically binding to an anti-ASFV antibody and with a different color from the color of the microspheres and/or nanospheres in the detector reagent specifically binding to an anti-CSFV antibody.

Commercially available microspheres have several sizes and polymers. Some considerations when selecting appropriate microspheres include the following:
- Optimal flow rate is achieved by choosing microspheres 1/10 the pore size of the membrane through which they will migrate, or smaller.
- Optimal colors for visualization in various types of samples.

To minimize hindered flow caused by the inherent hydrophobic interactions between membrane and particle (in the case of a hydrophobic membrane), pretreatment of the membrane with a substance that will maintain a small distance between the microspheres and the membrane, yet which can be easily rehydrated, is often helpful. Examples of substances commonly used are sucrose, various water soluble inert polymers, and surfactants. The idea is to choose a substance that is stable in dry form, yet dissolves easily upon rewetting to allow the antibody bound microspheres to flow easily through the membrane upon addition of the sample.

In addition to treating the membranes, the microspheres themselves can also be pre-treated with surfactants, synthetic or protein-based blockers. If done correctly, this can also help to reduce the problem of reagent mobility upon sample introduction. Much work has been done in developing optimum mixtures of these various polymers, detergents, and blockers.

Although any latex particle may be used in the present invention, the latex particles are typically formed from polystyrene, butadiene styrenes, styreneacrylic-vinyl terpolymer, polymethylmethacrylate, polyethylmethacrylate, styrene-maleic anhydride copolymer, polyvinyl acetate, polyvinylpyridine, polydivinylbenzene, polybutyleneterephthalate, acrylonitrile, vinylchloride-acrylates, and the like, or an aldehyde, carboxyl, amino, hydroxyl, or hydrazide derivative thereof.

In a particular embodiment of the invention, the detector reagent specifically binding to the anti-ASFV antibody, the detector reagent specifically binding to the anti-CSFV antibody and/or the detector reagent specifically binding to a control reagent is/are dehydrated or in dry form. In a particular embodiment, said detector reagents are reconstituted in contact with the test sample and/or in contact with a buffer solution.

In a particular preferred embodiment of the invention, the immunochromatographic strip of the invention comprises:
(i) a first set of particles, colored in a first color, dehydrated or in dry form and susceptible to be reconstituted by contacting with the test sample and/or a buffer solution, wherein said first particles are bound to, totally coated by or partially coated by the antigen, or fragment thereof, of the detector reagent specifically binding to an anti-ASFV antibody,
(ii) a second set of particles, colored in a second color, same as or different from said first color, dehydrated or in dry form and susceptible to be reconstituted by contacting with the test sample and/or a buffer solution, wherein said second particles are bound to, totally coated by or partially coated by the antigen, or fragment thereof, of the detector reagent specifically binding to the anti-CSFV antibody, and
(iii) a third set of particles, colored in a third color, same as or different from said first and/or second colors, optionally dehydrated or in dry form and susceptible to be reconstituted by contacting with the test sample, and/or a buffer solution, wherein said third particles are bound to, totally coated by or partially coated by a control reagent of the detector reagent.

In a particular embodiment, the first set of particles of the detector reagent specifically binding to an anti-ASFV antibody, are colored in a first color, the second set of particles of the detector reagent specifically binding to an anti-CSFV antibody, are colored in a second color, and the third set of particles of the detector reagent specifically binding to a control detector reagent, are colored in a third color, wherein said first, second and third colors are different colors from each other. In a particular embodiment, said particles are selected from the group comprising microspheres and nanospheres. In a more particular embodiment, said particles are selected from the group comprising latex microspheres, latex nanospheres, colloidal gold microspheres and colloidal gold nanospheres.

### 2.4 Signal detection pad (3)

A third element of the immunochromatographic strip according to the invention is the signal detection pad (3). In a particular embodiment, the signal detection pad (3) is located following the conjugate pad (2). In a particular embodiment, the signal detection pad (3) is coupled to the conjugate pad (2). In an alternative embodiment, as shown in Figure 1, the signal detection pad (3) is partially overlapping the conjugate pad (2).

In an embodiment, the signal detection pad (3) of the immunochromatographic strip of the invention comprises a signal detection zone. In a particular preferred embodiment, the signal detection pad (3) of the immunochromatographic strip of the invention comprises both a signal detection zone and a control zone. In a more particular embodiment, the signal detection pad (3) of the immunochromatographic strip of the invention comprises at least one signal detection zone, wherein said signal detection zone is selected from the group consisting of:
- an anti-ASFV signal detection zone (6), wherein the presence of an anti-ASFV antibody in the sample is detected, and wherein said signal detection zone comprises at least one capture reagent binding to an anti-ASFV antibody, wherein said at least one capture reagent is selected from the group consisting of an antigen, or a fragment thereof, recognized by said anti-ASFV antibody, and
- an anti-CSFV signal detection zone (5), wherein the presence of an anti-CSFV antibody in the sample is detected, and wherein said signal detection zone comprises at least one capture reagent binding to an anti-CSFV antibody, wherein said at least one capture reagent is selected from the group consisting of an antigen, or a fragment thereof, recognized by said anti-CSFV antibody.

In a particular preferred embodiment, the signal detection pad (3) of the immunochromatographic strip according to the present invention comprises both an anti-ASFV signal detection zone and an anti-CSFV signal detection zone. In a more preferred embodiment, the signal detection pad of the immunochromatographic strip according to the present invention comprises an anti-ASFV signal detection, an anti-CSFV signal detection zone and a control zone.

Additionally, the signal detection pad (3) of the immunochromatographic strip of the invention comprises a control zone (7), wherein said control zone (7) comprises a control capture reagent specifically binding to a control reagent, wherein the presence of a control reagent is detected if the test has been performed correctly, regardless of the presence of anti-ASFV antibodies and/or anti-CSFV antibodies in the sample.

The signal detection pad of the immunochromatographic strip of the invention is constituted by a membrane, wherein said membrane comprises the signal detection zone(s) (5, 6) and control zone (7) as described above.

As the skilled person acknowledges, physical and chemical properties of the membrane affect its capillary flow properties. The capillary flow properties in turn affect reagent deposition, assay sensitivity, assay specificity, and test line(s) consistency. In contrast, other physical properties of the membrane affect integration into finished test strips.

For lateral flow test strips, the membrane must irreversibly bind capture reagents at the test line(s) and control lines. The polymer from which the membrane is made determines most of its binding characteristics. If the membrane undergoes a secondary process that chemically alters the polymer or buries it under a second polymer, protein binding properties may be dramatically altered. Typical polymers include, without limitation, nitrocellulose (characterized by an electrostatic primary binding mechanism), polyvinylidene fluoride (characterized by an hydrophobic primary binding mechanism), nylon such as charge-modified nylon (characterized by an ionic/electrostatic primary binding mechanism) and polyethersulfone (characterized by an hydrophobic primary binding mechanism).

The protein binding capacity of a membrane is determined by the amount of polymer surface area available for immobilization. The surface area of a membrane is determined by pore size, porosity (amount of air in the three dimensional structure), thickness, and, to a minor extent, structural characteristics unique to the polymer. All other parameters being equal, surface area decreases nonlinearly with pore size, increases linearly with thickness, and increases nonlinearly with porosity.

The internal surface area of porous structures is normally reported as m²/g of polymer. For membranes, it is relatively easy to determine basis weight in grams/m². Multiplying the internal surface area by the basis weight results in the surface area ratio, expressed as m² internal surface area/m² frontal area. For membranes with very small pore sizes (about 0.1 µm), this ratio may exceed 2,000. In a particular embodiment, membranes used in immunochromatographic applications have surface area ratios in the range of 50 to 200.

The loading capacity of a protein on a given surface area depends on the compactness of structure of the protein and its Stokes radius (effective diameter). For IgG, the approximate loading capacity is 1 µg/cm². Multiplying the loading capacity of IgG (1 µg/cm²) by the surface area ratio of the membrane (50 - 200) produces an approximate IgG binding capacity of 50 - 200 µg/cm². In a typical immunochromatographic test strip, the test line is 1 mm wide. If the strip is 1 cm wide, the amount of capture reagent that can be bound is 5 - 20 µg (0.1 cm width x 1 cm long = 0.1 cm²). This is 10 - 100 times greater than required for most assays. Thus, protein binding capacity is normally not an issue in test design.

In a particular embodiment, the membrane of the signal detection pad (3) is a nitrocellulose membrane. Nitrocellulose membranes bind proteins electrostatically through interaction of the strong dipole of the nitrate ester with the strong dipole of the peptide bonds of the protein. Nitrocellulose membranes are completely neutral with no acidic protons. Although their ability to adsorb protein is independent of the pH of the immobilization solution, pH can affect the immobilization efficiency of a particular protein by altering its properties in solution. When applying capture reagents to nitrocellulose membranes, chaotropic agents such as Tween® 20 and Triton™ X-100 should be omitted or used at concentrations <0.01% v/v. These compounds can physically interfere with molecular contact between the protein and nitrocellulose. If chaotropic reagents are required to prevent non-specific interactions or reduce background, they should be put into the sample pad.

The capillary flow rate is the speed at which a sample front moves along a membrane strip when liquid is introduced at one end. Parameters affecting the capillary flow rate of a membrane are pore size, pore size distribution, porosity.

### 2.5 Capture reagents of the signal detection pad

Capture reagents are immobilized on the membrane of the signal detection pad. The capture reagent binding to an anti-ASFV antibody is immobilized on a first signal detection zone of the signal detection pad and the capture reagent binding to an anti-CSFV antibody is immobilized on a second signal detection zone of the signal detection pad, wherein said first and second signal detection zones are different, separated signal detection zones. In a particular embodiment, the signal detection zone wherein the capture reagent binding to an anti-ASFV antibody is immobilized is located closer to the conjugate pad than the signal detection zone wherein the capture reagent binding to an anti-CSFV antibody is immobilized. In an alternative embodiment, the signal detection zone wherein the capture reagent binding to an anti-CSFV antibody is immobilized is located closer to the conjugate pad than the signal detection zone wherein the capture reagent binding to an anti-ASFV antibody is immobilized.

In a particular embodiment, the capture reagent located in the anti-ASFV signal detection zone and specifically binding to an anti-ASFV antibody comprises the VP72 protein or a fragment thereof capable of binding to said anti-ASFV antibody.

In another particular embodiment, the capture reagent located in the anti-CSFV signal detection zone and specifically binding to an anti-CSFV antibody comprises the E2 protein or a fragment thereof capable of binding to said anti-CSFV antibody.

In a particular embodiment, the control capture reagent located in the control zone and specifically binding to a control reagent is selected from the group comprising a protein, or an antibody specifically binding to said control reagent. In a particular embodiment of the invention, the capture reagent specifically binding to the anti-ASFV antibody, the capture reagent specifically binding to the anti-CSFV antibody and the capture reagent specifically binding to a control reagent are dehydrated or in dry form. In a particular embodiment, said capture reagents are reconstituted in contact with the test sample and/or in contact with a buffer solution.

The buffers used for dilution of the capture reagents have to be optimized so that protein adsorption is not compromised, reagent reactivity is retained, and flow properties of the membrane are not altered.

Capture reagent solutions should always be buffered to achieve consistency of manufacturing and test strip performance. Buffers that can be used to control the pH of capture reagent solutions include, without limitation, phosphate buffer saline (PBS), borate buffer saline (BBS), Tris-buffered saline, and carbonate buffer. When selecting the appropriate buffer, it must be taken into account that the capture reagent is dried onto the membrane in the presence of the buffer salts. Problems can arise from chemical interactions that occur during evaporation, when the concentration of these salts becomes transiently very high. For example, if the reagent solution is buffered with a primary amine such as TRIS or glycine, salt bridging can occur between acidic amino acid residues (glutamic acid and aspartic acid) in the capture reagent and the -NH3⁺ group of the buffer molecule, reducing the ability of the capture reagent to bind the analyte. Sodium is a better cation than potassium for the same reason. For capture reagents that are amenable to a pH range of 4 to 6, ammonium acetate can be used.

In relation to the pH of the capture reagent buffer, it must be selected appropriately in order to minimize protein solubility and maintain a stable solution, which is usually achieved adjusting the pH to ±1 unit of the pI of the capture reagent.

The ionic strength of the capture reagent buffer should be reduced as much as possible. Ions in solution can interfere with electrostatic interactions between the membrane and the capture reagents. In addition, physiological concentrations of buffer salts and sodium chloride promote the solubility of most proteins and reduce the hydrophobic attraction of the nitrocellulose membrane. Finally, as the molarity of the capture reagent solution increases, the amount of solid residue left on the membrane after evaporation also increases. Solid residue in the pores can delay wetting of the membrane and alter its flow properties when the test is run. Thus, the molarity of the buffer should be lowered to the minimum required to maintain a stable pH (usually <10 mM). Sodium chloride should be eliminated unless absolutely required for protein solubility or stability.

The addition of a small amount of alcohol (1% to 10% v/v methanol, ethanol or isopropanol) to the reagent application solution can be extremely beneficial for three distinct reasons. First, the alcohol can dramatically improve the consistency of reagent application by lowering solution viscosity, by lowering solution surface tension (both of which promote entry of the solution into the membrane), and by decreasing static repulsion. Second, the addition of a small amount of alcohol decreases protein solubility without causing denaturation or precipitation. It also promotes the adsorption of the protein onto the nitrocellulose membrane. Third, the presence of even a low concentration of alcohol enhances drying and protein fixation. The benefits of adding alcohol should be determined empirically by the skilled person.

Occasionally, the addition of a capture reagent solution displaces surfactant present in the membrane, causing the reagent line to wet more slowly than adjacent areas of untreated membrane. When this occurs, the flow front becomes deformed. In extreme cases, the flow front wraps around the capture reagent line, entrapping air (especially when the membrane is sandwiched between two layers of nonporous plastic) and preventing flow over the line. To prevent this, it is sometimes effective to add a small amount of detergent (e.g., 0.05% SDS or 0.005% Triton™ X-100) to promote rewetting of the line.

### 2.6 Absorbent pad (4)

A fourth element of the immunochromatographic strip according to the invention is the absorbent pad (4). In a particular embodiment, the absorbent pad (4) is located following the signal detection pad (3). In a more particular embodiment the absorbent pad (4) is coupled to the signal detection pad (3). In a particular preferred embodiment, as shown in Figure 1, the absorbent pad (4) is partially overlapping the signal detection pad (3).

The absorbent pad is typically placed at the distal end of the test strip. The primary function of the absorbent pad is to increase the total volume of sample that enters the test strip. This increased volume can be used to wash unbound detector particles away from the signal detection zone(s) (test line(s) and control zone (control line), thereby lowering the background and enhancing assay sensitivity. Since the volume of sample that ultimately contributes to signal is controlled by the volume required to solubilize the detector particles, and not by the total volume of sample that enters the device, the addition of the absorbent pad may not have a dramatic impact on overall assay sensitivity.

Most absorbent pads are made from cellulose filters. The material should be selected on the basis of thickness, compressibility, manufacturability, and, most of all, uniformity of bed volume. Once an absorbent material has been chosen, optimizing the overall volume uptake for the test strip is best managed by changing the dimensions (usually the length) of the absorbent pad.

### 2.7 Additional/Optional elements of the immunochromatographic strip of the invention

Additional, optional, components of the immuchromatographic strip of the invention include a backing card (9) (such as a plastic backing card or a plastic-adhesive backing card), a protector adhesive (8), a cover tape (such as a laminate cover tape) and a strip housing cassette.

Due to the delicate nature of the materials used in an immunochromatographic strip assay as well as the need to maintain a precise, direct contact between components to ensure proper reagent and sample flow a backing card of some sort is adequate. Usually these are made pre-treated with pressure-sensitive adhesive selected for its stability in the assay and to insure it does not leach chemicals that may interfere with results. A related concern is that the adhesive is strong enough to properly bind the materials to the card but that it also does not flow too far in to them and inhibit the capillary action by reducing the available bed volume. The adhesive card is initially covered with a liner which may be pre-slit for easier assembly of test components. Suitable materials include, without limitation, polystyrene, vinyl, polyester (clear or opaque), and Mylar.

The laminate cover tape is an adhesive tape the acts as a protective barrier and prevents evaporation of reagents and helps to limit back-flow of reagents. When using some particularly delicate materials a cover tape is essential to maintain integrity of the test. The tape may have any number of designs imprinted upon it, such as test identification or trade names. For obvious reasons the cover tape must be clear over the test and control line sections of the immunochromatographic strip assay. A fine balance must be achieved between adhesive strength and migration of the adhesives to prevent assay interference and loss of bed-volume.

A plastic housing typically made of two pieces that snap together and protect the assembly. The test strip and absorbent pad are contained within this housing that allows the unit to be handheld more easily and protects the strip from damage and environmental contamination. A window on the side of the housing allows the test and control zone portions of the strip to be viewed. In some instances this is little more than a hole in one half of the cassette but it may also be a clear plastic window that protects the membrane from being accidentally damaged or splashed while still allowing visualization of the strip.

The immunochromatographic strips of the invention may additionally be stored and/or sealed in suitable containers including, without limitation, aluminium bags, plastic bags, and suitable containers known by the skilled person.

The immunochromatographic strip of the invention can be used for detecting anti-ASFV antibodies and/or anti-CSFV antibodies in a sample, or for diagnosing an infection caused by ASFV and/or by CSFV, or for discriminating between ASFV and CSFV as the causative agent of an infection in a subject.

### 3. Kits of the invention and uses thereof

In a further aspect, the present invention relates to a kit (hereinafter referred to as "kit of the invention") comprising at least one immunochromatographic strip according to the invention, which has been previously described.

In the present invention, a "kit" is understood as a product comprising different reagents and materials for determining the presence or the absence of anti-ASFV antibodies and/or anti-CSFV antibodies in a sample from a subject. Said kit comprises at least one immunochromatographic strip according to the invention, which has been described previously.

In a particular embodiment, the immunochromatographic strip of the kit is in a simplex format, that is to say, comprising a single strip. In an alternative particular embodiment, the immunochromatographic strip of the kit is in a multiplex format, that is to say, the strip is in a 2-strip format, a 3-strip format, a 4-strip format, a 5-strip format, a 6-strip format, a 7-strip format, a 8-strip format of even more. Said 2-strip format, 3-strip format, 4-strip format, 5-strip format, 6-strip format, 7-strip format, 8-strip and 12-strip format is suitable for dipping said strip format into a plate, such as 6-well plate, 24-well plate or 96-well plate. Preferably, the immunochromatographic strip of the kit is in a 8-strip format, suitable for dipping said 8-strip format into a 96-well plate.

Another component which can be present in the kit is a packing which allows maintaining the immunochromatographic strip(s) properly stored. Suitable materials for preparing such packagings include glass, plastic (polyethylene, polypropylene, polycarbonate and the like), bottles, vials, pipettes, paper, sachets and the like. The kit of the invention can additionally contain instructions for using the immunochromatographic strip(s) of the invention and/or instructions for using additional agents necessary to develop a lateral flow immunochromatography by means of said immunochromatographic strip(s) of the invention. Said instructions can be found in the form of printed material or in the form of an electronic support which can store instructions such that they can be read by a subject, such as electronic storage media (magnetic disks, tapes and the like), optical media (CD-ROM, DVD) and the like. The media can additionally or alternatively contain internet websites providing said instructions.

In an additional aspect, the invention relates to the use of the kit of the invention as previously described for detecting anti-ASFV antibodies and/or anti-CSFV antibodies, for example, in a sample from a subject; or for diagnosing an infection caused by ASFV and/or by CSFV, or for discriminating between ASFV and CSFV as the causative agent of an infection in a subject. In a particular embodiment, the use of the kit of the invention for detecting anti-ASFV antibodies and/or anti-CSFV antibodies, or for diagnosing an infection caused by ASFV and/or by CSFV, or for discriminating between ASFV and CSFV as the causative agent of an infection in a subject is performed by an immunoassay based on lateral flow chromatography.

### 4. Immunoassay methods of the invention

In another aspect, the invention relates to an immunoassay method (hereinafter referred to as "immunoassay method of the invention") for detecting the presence of anti-ASFV antibodies and/or anti-CSFV antibodies in a subject, wherein said immunoassay method is performed by means of an immunochromatographic strip of the invention, said method comprising
(i) contacting a sample from said subject with a sample pad of the immunochromatographic strip,
(ii) incubating under suitable conditions for the formation of complexes in the conjugation pad of said immunochromatographic strip between the anti-ASFV antibody and a detector reagent specifically binding to said anti-ASFV antibody and/or complexes between the anti-CSFV and a detector reagent specifically binding to said anti-CSFV antibody, and the migration of said complexes to the signal detection pad of the immunochoromatographic strip, and
(iii) detecting in the signal detection zone of said immunochromatographic strip the formation of complexes between the anti-ASFV antibody-detector reagent and a capture reagent specifically binding to said anti-ASFV antibody and/or the complexes between the anti-CSFV-detector reagent and a capture reagent specifically binding to said anti-CSFV antibody,
wherein the detection of said complexes between the anti-ASFV antibody and a capture reagent specifically binding to said anti-ASFV antibody is indicative of the presence of anti-ASFV antibodies in said subject, and/or the detection of said complexes between the anti-CSFV antibody and a capture reagent specifically binding to said anti-CSFV antibody is indicative of the presence of anti-CSFV antibodies in said subject.

Thus, in a first step, the method of the invention comprises contacting a sample from a subject with a sample pad of the immunochromatographic strip of the invention, previously described.

In a particular embodiment, the subject is a mammal, particularly a swine, whose presence or absence of anti-ASFV antibodies and/or anti-CSFV antibodies, indicative of an infection by ASFV or CSFV, respectively, is to be determined. Samples according to the invention have been described previously. In a particular embodiment, the sample from a subject to be analyzed by the immunoassay method of the invention is selected from the group comprising whole blood, serum, plasma, meat juices and saliva. In a particular embodiment, the subject is alive. In a particular alternative embodiment, the subject is deceased.

The volume of the sample from a subject which is put into contact with the sample pad of the strip, as the skilled person understands, must allow the formation of complexes between the anti-ASFV antibodies and/or anti-CSFV antibodies, when present in the sample, and the detector reagent of the strip in quantity enough to be captured by corresponding capture reagents and detected. An exemplary sample volume is comprised between 5 and 100 µl.

As the skilled person understands, the sample to be analyzed can be contacted with the sample pad of the strip by dropping said sample on the sample pad or by dipping the sample pad of the strip in a container comprising a sample, wherein said container is selected from the group comprising test tubes, culture plates, vials, and any other sample container known by the skilled person.

In a second step, the immunoassay method of the invention comprises incubating under suitable conditions for the formation of complexes in the conjugation pad of said immunochromatographic strip between the anti-ASFV antibody and a detector reagent specifically binding to said anti-ASFV antibody and/or the complexes between the anti-CSFV and a detector reagent specifically binding to said anti-CSFV antibody, and the migration of said complexes to the signal detection pad. Suitable conditions for the formation of the complexes between the antibodies and corresponding detector regions, and for the subsequent formation of complexes with a capture region include adequate conditions of temperature, humidity, etc., which can be determined routinely by the skilled person.

In a third step, the immunoassay method of the invention comprises detecting in the signal detection zone of said immunochromatographic strip the formation of complexes between the anti-ASFV antibody-detector reagent and a capture reagent specifically binding to said anti-ASFV antibody and/or the complexes between the anti-CSFV-detector reagent and a capture reagent specifically binding to said anti-CSFV antibody.

As previously described, the signal detection pad of the immunochromatographic strip of the invention comprises a signal detection zone, comprising at least one capture reagent specifically binding to an anti-ASFV antibody and/or at least one capture reagent specifically binding to an anti-ASFV antibody, and a control zone, comprising a control capture reagent specifically binding to a control reagent.

If the sample under analysis comprises anti-ASFV antibodies, a complex between said anti-ASFV antibodies and the detector reagent specifically binding to anti-ASFV antibodies is formed, and said complex migrates along the membrane until reaching the anti-ASFV signal detection zone, wherein the capture reagent binding to anti-ASFV antibodies binds to and retains the complex. Therefore, this complex is visualized or detected since, as previously described, the detector reagent of the complex is detectable visually or by an instrumental device. Therefore, detection of a complex between the anti-ASFV antibody and a capture reagent specifically binding to said anti-ASFV antibody is indicative of the presence of anti-ASFV antibodies in a subject. Similarly, detection of a complex between the anti-ASFV antibody and a capture reagent specifically binding to said anti-ASFV antibody is indicative of infection by ASFV in a subject.

Similarly, if the sample under analysis comprises anti-CSFV antibodies, a complex between said anti-CSFV antibodies and the detector reagent specifically binding to anti-CSFV antibodies is formed, and said complex migrates along the membrane until reaching the anti-CSFV signal detection zone, wherein the capture reagent binding to anti-CSFV antibodies binds to and retains the complex. This complex is visualized or detected as well since, as previously described, the detector reagent of the complex comprises a probe which is detectable visually or by an instrumental device. Therefore, detection of a complex between the anti-CSFV antibody and a capture reagent specifically binding to said anti-CSFV antibody is indicative of the presence of anti-CSFV antibodies in a subject. Similarly, detection of a complex between the anti-CSFV antibody and a capture reagent specifically binding to said anti-CSFV antibody is indicative of infection by CSFV in a subject.

In a particular preferred embodiment, the immunochromatographic strip of the invention comprises a control reagent, a control detector reagent specifically binding to said control reagent, and a capture reagent specifically binding to said control reagent. If the immunochromatography develops appropriately, that is to say, the sample front flows along the strip, (so the analytes of the sample flow through the conjugate pad, the complexes of said analytes and their corresponding detector reagents flow through the detection pad until reaching the signal detection zone of said detection pad and are retained by their corresponding capture reagents), then the control reagent flows as well with the migration front and a complex of said control reagent and the control detector reagent flows through the conjugate pad, the complex of the control reagent and the control detector reagent flows through the signal detection pad until reaching the control detection zone of the detection pad, wherein said control detection zone is located afterwards the signal detection zone. During a test by the method of the invention, if no signal is detected in the control zone of said strip, a new test must be performed following the method of the invention by means of a new strip. In an alternative embodiment, the immunochromatographic strip of the invention comprises a control reagent and a capture reagent, wherein said control reagent comprises a label, particularly wherein said control reagent comprises colored particles to which the control reagent is bond to, or alternatively, to which the control reagent is totally or partially coating.

In a particular embodiment, the time interval between steps (i) and (iii) of the method of the invention is from 2 to 45 minutes, preferably from 5 to 30 minutes, more preferably from 5 to 20 minutes, even more preferably of approximately 10 minutes.

According to the immunoassay method of the invention, the detection of complexes between the anti-ASFV antibody and a capture reagent specifically binding to said anti-ASFV antibody in the anti-ASFV detection zone of the strip is indicative of the presence of anti-ASFV antibodies in said subject. Additionally or alternatively, the detection of complexes between the anti-CSFV antibody and a capture reagent specifically binding to said anti-CSFV antibody in the anti-CSFV detection zone of the strip is indicative of the presence of anti-CSFV antibodies in said subject.

In another aspect, the invention relates to an immunoassay method for discriminating between the causative agent of an infection in a subject, wherein said causative agent is selected from the group consisting of ASFV, CSFV and combinations thereof, wherein said immunoassay method is performed by means of the above mentioned immunochromatographic strip provided the invention, said method comprising
(i) contacting a sample from said subject with a sample pad of the immunochromatographic strip,
(ii) incubating under suitable conditions for the formation of complexes in the conjugation pad of said immunochromatographic strip between the anti-ASFV antibody and a detector reagent specifically binding to said anti-ASFV antibody and/or complexes between the anti-CSFV and a detector reagent specifically binding to said anti-CSFV antibody, and the migration of said complexes to the signal detection pad of the immunochoromatographic strip, and
(iii) detecting in the signal detection zone of said immunochromatographic strip the formation of complexes between the anti-ASFV antibody-detector reagent and a capture reagent specifically binding to said anti-ASFV antibody and/or the complexes between the anti-CSFV-detector reagent and a capture reagent specifically binding to said anti-CSFV antibody,
wherein the detection of said complexes between the anti-ASFV antibody and a capture reagent specifically binding to said anti-ASFV antibody is indicative of the presence of anti-ASFV antibodies in said subject, and the presence of said anti-ASFV antibodies is indicative that the causative agent of said infection is ASFV; and/or
wherein the detection of said complexes between the anti-CSFV antibody and a capture reagent specifically binding to said anti-CSFV antibody is indicative of the presence of anti-CSFV antibodies in said subject, and the presence of said anti-CSFV antibodies is indicative that the causative agent of said infection is CSFV.

The particulars and conditions for performing this immunoassay method are the same as those of the immunoassay method of the invention which are herein incorporated.

According to this immunoassay method of the invention, the detection of complexes between the anti-ASFV antibody and a capture reagent specifically binding to said anti-ASFV antibody in the anti-ASFV detection zone of the strip is indicative of the presence of anti-ASFV antibodies in said subject, and, consequently, the presence of said anti-ASFV antibodies is indicative that the causative agent of said infection is ASFV.

Additionally or alternatively, the detection of complexes between the anti-CSFV antibody and a capture reagent specifically binding to said anti-CSFV antibody in the anti-CSFV detection zone of the strip is indicative of the presence of anti-CSFV antibodies in said subject, and, consequently, the presence of said anti-CSFV antibodies is indicative that the causative agent of said infection is CSFV.

In a particular embodiment, the causative agent is ASFV. In another particular embodiment, the causative agent is CSFV. In another particular embodiment, the causative agent could be ASFV and CSFV.

The invention is detailed below by means of the following examples which are merely illustrative and by no means limiting the scope of the invention.

### EXAMPLE 1

### Immunochromatography-based test for detecting the presence of specific anti-ASFV or anti-CSFV antibodies in a sample

The inventors of the present invention have developed an immunochromatography-based test able to detect the presence in a sample from a subject of specific anti-ASFV and/or anti-CSFV antibodies.

The test comprises the use of three types of microspheres: blue microspheres which are covalently linked to E2 protein of CSFV, red microspheres which are covalently linked to VP72 protein of ASFV, and green microspheres which are linked to a control reagent and are used as test control.

On the membrane, two test lines are printed: T1 formed by the E2 protein of CSFV and T2 formed by the VP72 protein of ASFV. Furthermore, the test contains a control line formed by a specific monoclonal antibody (mAb) for the control reagent (biotin).

The antibodies present in the sample react with the latex particles which are coated with specific target proteins. This complex flows through the membrane. Depending on the type of antibody present in the sample, different colored lines appear. These lines will be used to interpret the test results. Interpretation of results should be done as follows 10 minutes after the addition of the sample.

If both a green line and a second pink/red line appear in the result area, it means an ASFV-positive result. Color intensity may vary depending on the antibody concentration.

If both a green line and a second blue line appear in the result area, it means a CSFV-positive result. Color intensity may vary depending on the antibody concentration.

If a single green line appears in the result area, it means a negative result. The control line, in this case the green line, should always appear.

If no green line appears, the test is considered to be invalid, and must be repeated using a new device. Additionally, no results must be considered after 10 minutes.

An scheme of the chromatographic strip according to the invention is shown in Figure 1.

### 1.1 Materials & Methods

### Capture reagents

CSFV E2 protein and ASFV VP72 protein were used as test line T1 and T2 capture reagents, respectively. The VP72 protein was diluted at 50 µg/ml in Tris/HCl 20 mM buffer at pH 7.5 containing 5% sucrose and 0.095% sodium azide as preservative. The E2 protein was diluted at 150 µg/ml in the same buffer. The anti-biotin IgG monoclonal antibody was used as the control line capture reagent. It was diluted at 1 mg/ml in the same buffer used for the test lines. The aim of the control line was to confirm the success of the test.

The test and control capture reagents were dispensed in three parallel lines on 25 x 300 mm HiFlow Plus nitrocellulose membrane (HF120, Millipore) at 1 µl/cm. After drying for 5 minutes at 45°C, the membranes were sealed and stored at room temperature under dry conditions.

### Preparation of latex microparticle conjugated

The detector reagents consisted of 300 nm colored carboxyl-modified latex microspheres (PolymerLabs). The VP72 protein was covalently conjugated to red latex particles while the E2 protein was linked to blue latex particles. Finally, for the control latex, green particles were used to bind biotin. The proteins were conjugated as follows.

The particles (10% solids) were 10-fold diluted in coupling buffer (MES 10 mM pH 6.0) and centrifuged for 15 minutes at 12.000 rpm. The pellet was resuspended in MES buffer to 0.5% solids. To activate the surface, EDC (1-ethyl-3-[3-dimethylaminopropyl]carbodiimide hydrochloride) 1 mM and NHS (N-hydroxysuccinimide) 1 mM were simultaneously added to the latex and incubated for 45°C at room temperature with continuous mixing. The activated microspheres were centrifuged and resuspended again in MES (2-(N-morpholino)ethanesulfonic acid) buffer at 1% solids. The proteins were diluted in coupling buffer at 0.5 mg/m² and the activated latex was added. The mixture was incubated at room temperature for 2 hours with constant mixing. To inactivate the surface 500 mM imidazol was added and incubated for 15 minutes. Finally, the conjugated microspheres were washed with 0.1% Tween® 20 for 30 minutes. After this, the latex particles were centrifuged (15 minutes at 12.000 rpm), the supernatant was discarded and the pellet was resuspended in Tris/HCl 10 mM pH 8.2. The three conjugates were obtained following the same procedure. The microsphere solutions were stored at 4°C before use.

### Preparation of the conjugate pad

To prepare the conjugate solution, the VP72-latex, E2-latex and biotin-latex previously prepared were diluted to 0.2%, 0.2% and 0.15% respectively, in a Tris/HCl 25mM pH 9.5 buffer containing 35% sucrose, 1% Tween 20, 1.5% casein, 3% BSA (bovine serum albumin) and 0.095% sodium azide. The mixture was dispensed to the rayon conjugate pad (M1546, 10 x 300 mm, Ahlstrom) at 9 µl/cm. The pads were dried for 30 minutes at 45°C and stored at room temperature under dry conditions.

### Preparation of immunochromatographic strips

A master card was assembled as following: on a plastic backing with adhesive (76 x 300 mm), nitrocellulose membrane (25 x 300 mm), conjugated pad (10 x 300 mm), sample pad (Cytosep 1662, 25 x 300 mm) and absorbent pad (paper 237 grade, 29.5 x 300 mm) were pasted and covered with a protector film (65 x 300 mm). The master card was cut to 4.2 nm width strips. Trips were then put in a plastic house and sealed in an aluminium bag in the presence of desiccant gel and stored at room temperature.

### Test procedure

10 µl of the serum sample (plasma, whole blood) were added on the sample pad and 3-4 drops of running buffer (Tris/HCl pH 7.5, NaCl, casein and 0.09% NaN₃ as preservative) were applied.

### 1.2 Results

Different parameters have been evaluated to determine the accuracy of the assay. It is important to remark that the present test is a qualitative assay, resulting in a positive or negative decision.

The analytical sensitivity has been evaluated using specific monoclonal antibodies to the target proteins used in the test (VP72-ASFV and E2-CSFV). A specific anti-ASFV monoclonal antibody (mAb) (18BG3, Vidal MI & Oliva AG 1997 Sensors and Actuators B 38-39: 448-451) was used to determine the sensitivity for the ASFV test line and a specific anti-CSFV monoclonal Ab (18.4) was used for the CSFV test line. A mixture of both antibodies diluted in the running buffer, at decreasing concentrations, was used. Each dilution was treated as an independent sample. As shown in Table 1, the minimum amount of anti-ASFV MAb able to be detected by the test was about 8 ng/ml or 0.96 ng per test, keeping in mind that the added volume to carry out the test was 120 µl. Regarding to the CSFV test line, the minimum amount of specific antibody detected was 125 ng/ml (15 ng/test).

**Table 1**

| **Analytical sensitivity for the duplex test using specific monoclonal antibodies (mAb)** | | | | | | | | |
|---|---|---|---|---|---|---|---|---|
| | **[mAb] (µg/ml)** | | | | | | | |
| | 1.000 | 0.500 | 0.250 | 0.125 | 0.063 | 0.031 | 0.016 | 0.008 |
| **ASFV test line** | positive | positive | positive | positive | positive | positive | weak positive | weak positive |
| **CSFV test line** | positive | positive | positive | positive | weak positive | doubtful | negative | negative |

In order to determine the diagnostic sensitivity, two panels of sera have been evaluated. On the one hand, 21 serum samples from ASFV experimental infections were used to determine the specificity for the ASFV test, and on the other hand, 22 serum samples from CSFV experimental infections were used to determine the sensitivity of the CSFV line. All serum samples used were from porcine origin. The first panel of sera consists of 14 positive (2 samples reported as weak positive) and 7 negative sera for ASFV. The positivity of these samples was determined using the commercial competition ELISA 11.PPA.K3 (Ingezim PP Compac) as reference test. 11 of 14 positive sera were positive for the ASFV line, and 3 of them showed a weak positive result. The 7 serum samples which were negative with the reference test were reported also as negative with the ASFV test. None of the 21 serum samples presented a positive result for the CSFV test line. Regarding the second panel of sera used on the evaluation of the immunochromatographic strip of the invention (identified by the inventors as "Duplex Lateral Flow Device" or simply "Duplex LFD"), all of 22 sera were assayed with a reference ELISA test and all of them showed a positive result. 20 of 22 sera showed a positive result for the CSFV test line, meanwhile 2 of them showed a weak positive result. No positive results were observed for the ASFV test line. The results of the evaluation of these serum samples with the immunochromatographic strip of the invention (Duplex LFD test) are shown in Tables 2 and 3 (2A, 2B).

**Table 2**

| **Qualitative results of the evaluation of the Duplex LFD test with samples from ASFV experimental infections** | | | |
|---|---|---|---|
| **Sample ID** | **Reference Test** | **ASFV line** | **CSFV line** |
| 1 | positive | positive | negative |
| 2 | positive | positive | negative |
| 3 | positive | positive | negative |
| 4 | positive | weak positive | negative |
| 5 weak | positive | positive | negative |
| 6 | positive | positive | negative |
| 7 | Positive | positive | Negative |
| 8 | Positive | weak positive | Negative |
| 16 | Positive | positive | Negative |
| 17 weak | Positive | positive | Negative |
| 18 | Positive | positive | Negative |
| 19 | Positive | positive | Negative |
| 22 | Positive | weak positive | Negative |
| 10 | Positive | positive | Negative |
| 11 | Negative | negative | Negative |
| 12 | Negative | negative | Negative |
| 13 | Negative | negative | Negative |
| 14 | Negative | negative | Negative |
| 15 | Negative | negative | Negative |
| 20 | Negative | negative | Negative |
| 21 | Negative | negative | Negative |

**Table 3**

| **Qualitative results of the evaluation of the Duplex LFD test with samples from CSFV experimental infections** | | | |
|---|---|---|---|
| **Sample ID** | **Reference Test** | **ASFV line** | **CSFV line** |
| 1 | Positive | negative | positive |
| 2 | Positive | negative | positive |
| 3 | Positive | negative | positive |
| 4 | Positive | negative | positive |
| 5 | Positive | negative | positive |
| 6 | Positive | negative | positive |
| 7 | Positive | negative | positive |
| 9 | Positive | negative | positive |
| 10 | Positive | negative | positive |
| 11 | Positive | negative | positive |
| 12 | Positive | negative | positive |
| 13 | Positive | negative | positive |
| 14 | Positive | negative | positive |
| 17 | Positive | negative | positive |
| 18 | Positive | negative | positive |
| 19 | Positive | negative | positive |
| 20 | Positive | negative | positive |
| 21 | Positive | negative | weak positive |
| 22 | Positive | negative | positive |
| 23 | Positive | negative | weak positive |
| 26 | Positive | negative | positive |
| 28 | Positive | negative | positive |

Analytical specificity: No cross-reaction between ASFV and CSFV was observed for any of the investigated serum samples, since each colloid recognizes specifically the antibodies present in the sample. Positive tests showed good lines, blue for the CSFV positive samples and red for the positive ASFV serum samples. On the other hand, 5 positive serum samples for other *Pestivirus* (BVDV and BDV) were analyzed and no cross-reactivity was observed for any of them.

Moreover, in order to determine the diagnostic specificity 50 serum samples from ASFV and CSFV from virus-free geographical areas were also tested. All samples showed a negative result for the new lateral flow device (LFD). In view of these results, an immunoassay test based on the immunochromatographic strips provided by this invention (Duplex LFD test) shows a diagnostic sensitivity higher than 99% and 100% of diagnostic specificity.

These results show that the now developed test based on the immunochromatographic strips provided by this invention (Duplex LFD test) provides a reliable immunoassay method for rapid detection of anti-ASFV and anti-CSFV antibodies.

## Claims

1. An immunochromatographic strip comprising
(i) a sample pad (1), wherein said sample pad receives the sample to be analyzed,
(ii) a conjugate pad (2), coupled to or partially overlapping the sample pad (1), wherein said conjugate pad comprises
- at least one detector reagent specifically binding to an anti-African swine fever virus (ASFV) antibody, wherein said at least one detector reagent comprises an antigen, or a fragment thereof, recognized by the anti-ASFV antibody,
- at least one detector reagent specifically binding to an anti-classical swine fever virus (CSFV) antibody, wherein said at least one detector reagent comprises an antigen, or a fragment thereof, recognized by the anti-CSFV antibody, and
- at least one detector reagent specifically binding to a control reagent,
(iii) a signal detection pad (3), coupled to or partially overlapping the conjugate pad (2), wherein said signal detection pad comprises
- at least one signal detection zone, consisting of:
• an anti-ASFV signal detection zone (6), wherein the presence or the absence of anti-ASFV antibodies in the sample is detected, and wherein said detection zone comprises at least one capture reagent specifically binding to an anti-ASFV antibody, wherein said at least one capture reagent is selected from the group consisting of an antigen, or a fragment thereof, recognized by said anti-ASFV antibody, and
• an anti-CSFV signal detection zone (5), wherein the presence or the absence of anti-CSFV antibodies in the sample is detected, and wherein said detection zone comprises at least one capture reagent specifically binding to an anti-CSFV antibody, wherein said at least one capture reagent is selected from the group consisting of an antigen, or a fragment thereof, recognized by the said anti-CSFV antibody,
wherein said anti-ASFV signal detection zone (6) and anti-CSFV signal detection zone (5) are different signal detection zones, and
- a control zone (7), comprising a control capture reagent specifically binding to a control reagent, wherein the presence of a control reagent is detected if the test has been performed correctly, regardless of the presence of anti-ASFV antibodies and/or anti-CSFV antibodies in the sample,
and,
(iv) an absorbent pad (4), coupled to or partially overlapping the signal detection pad (3)
wherein the capture reagent and/or the detector reagent specifically binding to an anti-ASFV antibody comprise(s) the VP72 protein or a fragment thereof capable of binding to said anti-ASFV antibody, and
wherein the capture reagent and/or the detector reagent specifically binding to the anti-CSFV antibody comprise(s) the E2 protein or a fragment thereof capable of binding to said anti-CSFV antibody.

2. Immunochromatographic strip according to claim 1, wherein:
- the conjugate pad (2) in (ii) comprises at least one detector reagent specifically binding to an anti-ASFV antibody, at least one detector reagent specifically binding to an anti-CSFV antibody, and at least one detector reagent specifically binding to a control reagent, and
- the signal detection zone of the signal detection pad (3) in (iii) comprises an anti-ASFV detection zone (5), an anti-CSFV detection zone (6), and a control zone (7).

3. Immunochromatographic strip according to any of claims 1 or 2, wherein said capture reagent and/or the detector reagent specifically binding to the anti-ASFV antibody, said capture reagent and/or the detector reagent specifically binding to the anti-CSFV antibody and/or said capture reagent and/or the detector reagent specifically binding to a control reagent is/are dehydrated or in dry form.

4. Immunochromatographic strip according to any of claims 1 to 3, wherein said antigen or fragment thereof of the detector reagent specifically binding to the anti-ASFV antibody, said antigen or fragment thereof of the detector reagent specifically binding to the anti-CSFV antibody, and/or said control detector reagent, is bonded to a particle, or it is totally or partially, coating a particle, wherein said particle is comprised by said detector reagent, and wherein said particle is selected from the group comprising colored latex particles and colored colloidal gold particles.

5. Immunochromatographic strip according to claim 4, wherein said particles are dehydrated or in dry form.

6. Immunochromatographic strip according to any of claims 1 to 5, comprising:
(i) a first set of particles, colored in a first color, said particles being dehydrated or in dry form and susceptible to be reconstituted by contacting with the test sample and/or a buffer solution, wherein said first particles are bound to, totally coated by, or partially coated by the antigen, or fragment thereof, of the detector reagent specifically binding to an anti-ASFV antibody,
(ii) a second set of particles, colored in a second color, wherein said second color is the same as, or different from, said first color, preferably said second color is different from said first color, said particles being dehydrated or in dry form and susceptible to be reconstituted by contacting with the test sample and/or a buffer solution, wherein said second particles are bound to, totally coated by, or partially coated by the antigen, or fragment thereof, of the detector reagent specifically binding to the anti-CSFV antibody, and
(iii) a third set of particles, colored in a third color, wherein said third color is the same as, or different from, said first and/or second color, preferably said third color is different from said first color and from said second color, the first and second colors being also different each other, said particles being optionally dehydrated or in dry form and susceptible to be reconstituted by contacting with the test sample, and/or a buffer solution, wherein said third particles are bound to, totally coated by, or partially coated by a control reagent of the control detector reagent.

7. Immunochromatographic strip according to any of claims 1 to 6, wherein the control reagent is selected from the group comprising biotin, avidin, streptavidin, BSA, HSA, KLH and OVA, and the capture reagent specifically binding to said control reagent is selected from the group comprising anti-biotin antibody, avidin, streptavidin, anti-BSA antibody, anti-HSA antibody, anti-KLH antibody, and anti-OVA antibody.

8. A kit comprising at least one immunochromatographic strip according to any of claims 1 to 7.

9. The kit according to claim 8, wherein the immunochromatographic strip is in a simplex format or in a multiplex format.

10. Use of an immunochromatographic strip according to any of claims 1 to 7, or a kit according to claim 8 or 9, for detecting anti-ASFV antibodies and/or anti-CSFV antibodies in a sample, or for diagnosing an infection caused by ASFV and/or by CSFV, or for discriminating between ASFV and CSFV as the causative agent of an infection in a subject.

11. Use of an immunochromatographic strip or kit according to claim 10, wherein the detection of anti-ASFV antibodies and/or anti-CSFV antibodies, and/or the diagnosis of infection is performed by an immunoassay based on lateral flow chromatography.

12. An immunoassay method for detecting the presence of anti-ASFV antibodies and/or anti-CSFV antibodies in a subject, wherein said immunoassay method is performed by means of an immunochromatographic strip according to any of claims 1 to 7, said method comprising
(i) contacting a sample from said subject with a sample pad of the immunochromatographic strip,
(ii) incubating under suitable conditions for the formation of complexes in the conjugation pad of said immunochromatographic strip between the anti-ASFV antibody and a detector reagent specifically binding to said anti-ASFV antibody and/or complexes between the anti-CSFV and a detector reagent specifically binding to said anti-CSFV antibody, and the migration of said complexes to the signal detection pad of the immunochoromatographic strip, and
(iii) detecting in the signal detection zone of said immunochromatographic strip the formation of complexes between the anti-ASFV antibody-detector reagent and a capture reagent specifically binding to said anti-ASFV antibody and/or the complexes between the anti-CSFV-detector reagent and a capture reagent specifically binding to said anti-CSFV antibody,
wherein the detection of said complexes between the anti-ASFV antibody and a capture reagent specifically binding to said anti-ASFV antibody is indicative of the presence of anti-ASFV antibodies in said subject, and/or the detection of said complexes between the anti-CSFV antibody and a capture reagent specifically binding to said anti-CSFV antibody is indicative of the presence of anti-CSFV antibodies in said subject.

13. An immunoassay method for discriminating between the causative agent of an infection in a subject, wherein said causative agent is selected from the group consisting of ASFV, CSFV and combinations thereof, wherein said immunoassay method is performed by means of an immunochromatographic strip according to any of claims 1 to 7, said method comprising
(i) contacting a sample from said subject with a sample pad of the immunochromatographic strip,
(ii) incubating under suitable conditions for the formation of complexes in the conjugation pad of said immunochromatographic strip between the anti-ASFV antibody and a detector reagent specifically binding to said anti-ASFV antibody and/or complexes between the anti-CSFV and a detector reagent specifically binding to said anti-CSFV antibody, and the migration of said complexes to the signal detection pad of the immunochoromatographic strip, and
(iii) detecting in the signal detection zone of said immunochromatographic strip the formation of complexes between the anti-ASFV antibody-detector reagent and a capture reagent specifically binding to said anti-ASFV antibody and/or the complexes between the anti-CSFV-detector reagent and a capture reagent specifically binding to said anti-CSFV antibody,
wherein the detection of said complexes between the anti-ASFV antibody and a capture reagent specifically binding to said anti-ASFV antibody is indicative of the presence of anti-ASFV antibodies in said subject, and the presence of said anti-ASFV antibodies is indicative that the causative agent of said infection is ASFV; and/or
wherein the detection of said complexes between the anti-CSFV antibody and a capture reagent specifically binding to said anti-CSFV antibody is indicative of the presence of anti-CSFV antibodies in said subject, and the presence of said anti-CSFV antibodies is indicative that the causative agent of said infection is CSFV.

14. Immunoassay method according to the claim 12 or 13, wherein the sample is selected from the group comprising whole blood, serum, plasma, meat juices and saliva.

## Patentansprüche

1. Ein immunchromatographischer Streifen umfassend
(i) ein Probenkissen (1), wobei besagtes Probenkissen die zu analysierende Probe aufnimmt,
(ii) ein Konjugatkissen (2), verbunden oder teilweise überlappend mit dem Probenkissen (1), wobei besagtes Probenkissen umfasst
- mindestens ein Nachweisreagenz, das spezifisch an einen anti-Afrikanische Schweinepest-Virus (ASFV)-Antikörper bindet, wobei besagtes mindestens eine Nachweisreagenz ein Antigen oder ein Fragment davon umfasst, das durch den anti-ASFV-Antikörper erkannt wird,
- mindestens ein Nachweisreagenz, das spezifisch ein anti-klassische Schweinepest-Virus (CSFV)-Antikörper bindet, wobei besagtes mindestens eine Nachweisreagenz ein Antigen oder ein Fragment davon umfasst, das durch den anti-CSFV-Antikörper erkannt wird, und
- mindestens ein Nachweisreagenz, das spezifisch an ein Kontrollreagenz bindet,
(iii) ein Signaldetektionskissen (3), verbunden oder teilweise überlappend mit dem Konjugatkissen (2), wobei besagtes Signaldetektionskissen umfasst
- mindestens eine Signaldetektionszone, bestehend aus:
• einer anti-ASFV-Signaldetektionszone (6), wobei die Anwesenheit oder Abwesenheit von anti-ASFV-Antikörpern in der Probe detektiert wird, und wobei besagte Detektionszone mindestens ein Einfangreagenz umfasst, das spezifisch an einen anti-ASFV-Antikörper bindet, wobei besagtes mindestens ein Einfangreagenz ausgewählt ist aus der Gruppe bestehend aus einem Antigen oder einem Fragment davon, das von besagtem anti-ASFV-Antikörper erkannt wird, und
• einer anti-CSFV-Signaldetektionszone (5), wobei die Anwesenheit oder Abwesenheit von anti-CSFV-Antikörpern in der Probe detektiert wird, und wobei besagte Detektionszone mindestens ein Einfangreagenz umfasst, das spezifisch an einen anti-CSFV-Antikörper bindet, wobei besagtes mindestens ein Einfangreagenz ausgewählt ist aus der Gruppe bestehend aus einem Antigen, oder einem Fragment davon, das durch den anti-CSFV-Antikörper erkannt wird,
wobei besagte anti-ASFV-Signaldetektionszone (6) und anti-CSFV-Signaldetektionszone (5) unterschiedliche Signaldetektionszonen sind, und
- eine Kontrollzone (7), umfassend ein Kontrolleinfangreagenz, das spezifisch an ein Kontrollreagenz bindet, wobei die Anwesenheit eines Kontrollreagenz detektiert wird, falls der Test korrekt ausgeführt worden ist, ohne Rücksicht auf die Anwesenheit von anti-ASFV-Antikörpern und/oder anti-CSFV-Antikörpern in der Probe,
und,
(iv) ein Absorbenskissen (4), verbunden oder teilweise überlappend mit dem Signaldetektionskissen (3)
wobei das Einfangreagenz und/oder das Nachweisreagenz, das/die spezifisch an einen anti-ASFV-Antikörper bindet/binden, das VP72-Protein oder ein Fragment davon, das fähig ist besagten anti-ASFV-Antikörper zu binden, umfasst, und
wobei das Einfangreagenz und/oder das Nachweisreagenz, das/die spezifisch an einen anti-CSFV-Antikörper bindet/binden, das E2-Protein oder ein Fragment davon, das fähig ist besagten anti-CSFV-Antikörper zu binden, umfasst.

2. Ein immunchromatographischer Streifen gemäß Anspruch 1, wobei:
- das Konjugatkissen (2) in (ii) mindestens ein Nachweisreagenz, das spezifisch an einen anti-ASFV-Antikörper bindet, mindestens ein Nachweisreagenz, das spezifisch an einen anti-CSFV-Antikörper bindet, und mindestens ein Nachweisreagenz, das spezifisch an ein Kontrollreagenz bindet, umfasst, und
- die Signaldetektionszone des Signaldetektionskissens (3) in (iii) eine anti-ASFV-Detektionszone (5), eine anti-CSFV-Detektionszone (6), und eine Kontrollzone (7) umfasst.

3. Ein immunchromatographischer Streifen gemäß einem der Ansprüche 1 oder 2, wobei besagtes Einfangreagenz und/oder das Nachweisreagenz, die spezifisch an den anti-ASFV-Antikörper binden, besagtes Einfangreagenz und/oder Nachweisreagenz, die spezifisch an den anti-CSFV-Antikörper binden, und/oder besagtes Einfangreagenz und/oder Nachweisreagenz, die spezifisch an ein Kontrollreagenz binden, dehydriert oder in getrockneter Form ist/sind.

4. Ein immunchromatographischer Streifen gemäß einem der Ansprüche 1 bis 3, wobei besagtes Antigen oder Fragment davon des Nachweisreagenz, das spezifisch an den anti-ASFV-Antikörper bindet, besagtes Antigen oder Fragment davon des Nachweisreagenz, das spezifisch an den anti-CSFV-Antikörper bindet, und/oder besagtes Kontrollnachweisreagenz an ein Partikel gebunden ist/sind, oder vollständig oder teilweise ein Partikel beschichtet, wobei besagtes Partikel vom besagten Nachweisreagenz umfasst wird, und wobei besagtes Partikel aus der Gruppe umfassend gefärbte Latex-Partikel und gefärbte, kolloidale Goldpartikel ausgewählt ist.

5. Ein immunchromatographischer Streifen gemäß Anspruch 4, wobei besagte Partikel dehydriert oder in getrockneter Form sind.

6. Ein immunchromatographischer Streifen gemäß einem der Ansprüche 1 bis 5, umfassend:
(i) einen ersten Satz von Partikeln, in einer ersten Farbe gefärbt, wobei besagte Partikel dehydriert oder in trockener Form sind und für die Rekonstitution zugänglich sind durch das in Kontakt Bringen mit der Testprobe und/oder einer Pufferlösung, wobei besagte erste Partikel gebunden an, vollständig beschichtet durch, oder teilweise beschichtet durch das Antigen, oder ein Fragment davon, des Nachweisreagenz, das spezifisch an einen anti-ASFV-Antikörper bindet, sind,
(ii) einen zweiten Satz von Partikeln, in einer zweiten Farbe gefärbt, wobei besagte zweite Farbe die gleiche wie, oder verschieden von, besagte/r ersten Farbe, bevorzugt ist besagte zweite Farbe verschieden von besagter erster Farbe, wobei besagte Partikel dehydriert oder in trockener Form sind und für die Rekonstitution zugänglich sind durch das in Kontakt Bringen mit der Testprobe und/oder einer Pufferlösung, wobei besagte zweite Partikel gebunden an, vollständig beschichtet durch, oder teilweise beschichtet durch das Antigen, oder ein Fragment davon, des Nachweisreagenz, das spezifisch an einen anti-CSFV-Antikörper bindet, sind, und,
(iii) einen dritten Satz von Partikeln, in einer dritten Farbe gefärbt, wobei besagte dritte Farbe die gleiche wie, oder verschieden von, besagte/r ersten und/oder zweiten Farbe, bevorzugt ist besagte dritte Farbe verschieden von besagter erster und besagter zweiten Farbe, die erste und zweite Farbe sind ebenfalls verschiedenen voneinander, wobei besagte Partikel optional dehydriert oder in trockener Form sind und für die Rekonstitution zugänglich sind durch das in Kontakt Bringen mit der Testprobe, und/oder einer Pufferlösung, wobei besagte dritte Partikel gebunden an, vollständig beschichtet durch, oder teilweise beschichtet durch das Antigen, oder ein Fragment davon, des Nachweisreagenz, das spezifisch an ein Kontrollreagenz des Kontrollnachweisreagenz bindet, sind.

7. Ein immunchromatographischer Streifen gemäß einem der Ansprüche 1 bis 6, wobei das Kontrollreagenz ausgewählt ist aus der Gruppe umfassend Biotin, Avidin, Streptavidin, BSA, HSA, KLH und OVA, und das Einfangreagenz spezifisch bindet an besagtes Kontrollreagenz ausgewählt aus der Gruppe umfassend anti-Biotin-Antikörper, Avidin, Streptavidin, anti-BSA-Antikörper, anti-HSA-Antikörper, anti-KLH-Antikörper, und anti-OVA-Antikörper.

8. Ein Kit umfassend mindestens einen immunchromatographischen Streifen gemäß einem der Ansprüche 1 bis 7.

9. Das Kit gemäß Anspruch 8, wobei der immunchromatographische Streifen in einem Simplex-Format oder in einem Multiplex-Format ist.

10. Verwendung eines immunchromatographischen Streifens gemäß einem der Ansprüche 1 bis 7, oder eines Kits gemäß Anspruch 8 oder 9, zur Detektion von anti-ASFV-Antikörpern und/oder anti-CSFV-Antikörpern in einer Probe, oder zur Diagnose einer Infektion verursacht durch ASFV und/oder durch CSFV, oder zum Unterscheiden zwischen ASFV und CSFV als ursächliches Agens einer Infektion in einem Subjekt.

11. Verwendung eines immunchromatographischen Streifens oder Kits gemäß Anspruch 10, wobei die Detektion von anti-ASFV-Antikörpern und/oder anti-CSFV-Antikörpern, und/oder die Diagnose einer Infektion durch einen Immuntest basierend auf Lateralflusschromatographie durchgeführt wird.

12. Ein Immuntest-Verfahren zur Detektion der Anwesenheit von anti-ASFV-Antikörpern, und/oder anti-CSFV-Antikörpern in einem Subjekt, wobei besagtes Immuntest-Verfahren mittels eines immunchromatographischen Streifens gemäß einem der Ansprüche 1 bis 7 durchgeführt wird, wobei besagtes Verfahren umfasst
(i) in Kontakt Bringen einer Probe von besagtem Subjekt mit einem Probenkissen des immunchromatographischen Streifens,
(ii) Inkubieren unter geeigneten Bedingungen für die Bildung von Komplexen zwischen dem anti-ASFV-Antikörper und einem Nachweisreagenz, das spezifisch an besagten anti-ASFV-Antikörper bindet, und/oder Komplexe zwischen dem anti-CSFV-Antikörper und einem Nachweisreagenz, das spezifisch an besagten anti-CSFV-Antikörper bindet, im Konjugationskissen von besagtem immunchromatographischen Streifen und der Wanderung besagter Komplexe zum Signaldetektionskissen des immunchromatographischen Streifens, und
(iii) Detektieren der Bildung von Komplexen zwischen dem anti-ASFV-Antikörper-Nachweisreagenz und einem Einfangreagenz, das spezifisch an besagten anti-ASFV-Antikörper und/oder den Komplexen zwischen dem anti-CSFV-Nachweisreagenz und einem Einfangreagenz, das spezifisch an besagten anti-CSFV-Antikörper bindet, in der Signaldetektionszone von besagtem immunchromatographischen Streifen,
wobei die Detektion von besagten Komplexen zwischen dem anti-ASFV-Antikörper und einem Einfangreagenz, das spezifisch an besagte anti-ASFV-Antikörper bindet, die Anwesenheit von anti-ASFV-Antikörpern in besagtem Subjekt anzeigt, und/oder die Detektion von besagten Komplexen zwischen dem anti-CSFV-Antikörper und einem Einfangreagenz, das spezifisch an besagte anti-CSFV-Antikörper bindet, die Anwesenheit von anti-CSFV-Antikörpern in besagtem Subjekt anzeigt.

13. Ein Immuntest-Verfahren zur Unterscheidung zwischen dem ursächlichen Agens einer Infektion in einem Subjekt, wobei besagtes ursächliches Agens ausgewählt ist aus der Gruppe bestehend aus ASFV, CSFV und Kombinationen davon, wobei besagtes Immuntestverfahren mittels eines immunchromatographischen Streifens gemäß einem der Ansprüche 1 bis 7 ausgeführt wird, wobei besagtes Verfahren umfasst
(i) in Kontakt Bringen einer Probe von besagtem Subjekt mit einem Probenkissen des immunchromatographischen Streifens,
(ii) Inkubieren unter geeigneten Bedingungen für die Bildung von Komplexen im Konjugationskissen von besagtem immunchromatographischen Streifens zwischen dem anti-ASFV-Antikörper und einem Nachweisreagenz, das spezifisch an besagten anti-ASFV-Antikörper bindet, und/oder Komplexe zwischen dem anti-CSFV-Antikörper und einem Nachweisreagenz, das spezifisch an besagten anti-CSFV-Antikörper bindet, und der Wanderung besagter Komplexe zum Signaldetektionskissen des immunchromatographischen Streifens, und
(iii) Detektieren der Bildung von Komplexen zwischen dem anti-ASFV-Antikörper-Nachweisreagenz und einem Einfangreagenz, das spezifisch an besagten anti-ASFV-Antikörper und/oder Komplexen zwischen dem anti-CSFV-Nachweisreagenz und einem Einfangreagenz, das spezifisch an besagten anti-CSFV-Antikörper bindet, in der Signaldetektionszone von besagtem immunchromatographischen Streifen,
wobei die Detektion von besagten Komplexen zwischen dem anti-ASFV-Antikörper und einem Einfangreagenz, das spezifisch an besagten anti-ASFV-Antikörper bindet, die Anwesenheit von anti-ASFV-Antikörpern in besagtem Subjekt anzeigt, und die Anwesenheit von besagten anti-ASFV-Antikörpern anzeigt, dass das ursächliche Agens besagter Infektion ASFV ist; und/oder
wobei die Detektion von besagten Komplexen zwischen dem anti-CSFV-Antikörper und einem Einfangreagenz, das spezifisch an besagten anti-CSFV-Antikörper bindet, die Anwesenheit von anti-CSFV-Antikörpern in besagtem Subjekt anzeigt, und die Anwesenheit von besagten anti-CSFV-Antikörpern anzeigt, dass das ursächliche Agens besagter Infektion CSFV ist.

14. Ein Immuntestverfahren gemäß einem der Ansprüche 12 oder 13, wobei die Probe ausgewählt ist aus der Gruppe umfassend Vollblut, Serum, Plasma, Fleischsäfte und Speichel.

## Revendications

1. Bandelette d'immunochromatographie comprenant
(i) un tampon d'échantillon (1), où ledit tampon d'échantillon reçoit l'échantillon à analyser,
(ii) un tampon de conjugué (2), couplé à ou chevauchant partiellement le tampon d'échantillon (1), où ledit tampon de conjugué comprend
- au moins un réactif de détection qui se lie spécifiquement à un anticorps anti-virus de la peste porcine africaine (ASFV), où ledit au moins un réactif de détection comprend un antigène, ou un fragment de celui-ci, reconnu par l'anticorps anti-ASFV,
- au moins un réactif de détection qui se lie spécifiquement à un anticorps anti-virus de la peste porcine classique (CSFV), où ledit au moins un réactif de détection comprend un antigène, ou un fragment de celui-ci, reconnu par l'anticorps anti-CSFV, et
- au moins un réactif de détection qui se lie spécifiquement à un réactif de contrôle,
(iii) un tampon de détection de signal (3), couplé à ou chevauchant partiellement le tampon de conjugué (2), où ledit tampon de détection de signal comprend
- au moins une zone de détection de signal, consistant en :
• une zone de détection d'un signal anti-ASFV (6), où la présence ou l'absence d'anticorps anti-ASFV dans l'échantillon est détectée, et où ladite zone de détection comprend au moins un réactif de capture qui se lie spécifiquement à un anticorps anti-ASFV, où ledit au moins un réactif de capture est sélectionné dans le groupe consistant en un antigène, ou un fragment de celui-ci, reconnu par ledit anticorps anti-ASFV, et
• une zone de détection d'un signal anti-CSFV (5), où la présence ou l'absence d'anticorps anti-CSFV dans l'échantillon est détectée, et où ladite zone de détection comprend au moins un réactif de capture qui se lie spécifiquement à un anticorps anti-CSFV, où ledit au moins un réactif de capture est sélectionné dans le groupe consistant en un antigène, ou un fragment de celui-ci, reconnu par ledit anticorps anti-CSFV,
où lesdites zone de détection d'un signal anti-ASFV (6) et zone de détection d'un signal anti-CSFV (5) sont des zones de détection de signal différentes, et
- une zone de contrôle (7), comprenant un réactif de capture de contrôle qui se lie spécifiquement à un réactif de contrôle, où la présence d'un réactif de contrôle est détectée si le test a été réalisé correctement, sans tenir compte de la présence d'anticorps anti-ASFV et/ou d'anticorps anti-CSFV dans l'échantillon,
et,
(iv) un tampon absorbant (4), couplé à ou chevauchant partiellement le tampon de détection de signal (3)
où le réactif de capture et/ou le réactif de détection qui se lie/lient spécifiquement à un anticorps anti-ASFV comprend/comprennent la protéine VP72 ou un fragment de celle-ci capable de se lier audit anticorps anti-ASFV, et
où le réactif de capture et/ou le réactif de détection qui se lie/lient spécifiquement à l'anticorps anti-CSFV comprend/comprennent la protéine E2 ou un fragment de celle-ci capable de se lier audit anticorps anti-CSFV.

2. Bandelette d'immunochromatographie selon la revendication 1, dans laquelle :
- le tampon de conjugué (2) en (ii) comprend au moins un réactif de détection qui se lie spécifiquement à un anticorps anti-ASFV, au moins un réactif de détection qui se lie spécifiquement à un anticorps anti-CSFV, et au moins un réactif de détection qui se lie spécifiquement à un réactif de contrôle, et
- la zone de détection de signal du tampon de détection de signal (3) en (iii) comprend une zone de détection anti-ASFV (5), une zone de détection anti-CSFV (6), et une zone de contrôle (7).

3. Bandelette d'immunochromatographie selon l'une quelconque des revendications 1 ou 2, dans laquelle ledit réactif de capture et/ou le réactif de détection qui se lie spécifiquement à l'anticorps anti-ASFV, ledit réactif de capture et/ou le réactif de détection qui se lie spécifiquement à l'anticorps anti-CSFV et/ou ledit réactif de capture et/ou le réactif de détection qui se lie spécifiquement à un réactif de contrôle est/sont déshydraté(s) ou sous une forme sèche.

4. Bandelette d'immunochromatographie selon l'une quelconque des revendications 1 à 3, dans laquelle ledit antigène ou fragment de celui-ci du réactif de détection qui se lie spécifiquement à l'anticorps anti-ASFV, ledit antigène ou fragment de celui-ci du réactif de détection qui se lie spécifiquement à l'anticorps anti-CSFV, et/ou ledit réactif de détection de contrôle, est lié à une particule, ou est totalement ou partiellement revêtu sur une particule, où ladite particule est composée dudit réactif de détection, et où ladite particule est sélectionnée dans le groupe comprenant des particules de latex colorées et des particules d'or colloïdal colorées.

5. Bandelette d'immunochromatographie selon la revendication 4, dans laquelle lesdites particules sont déshydratées ou sous une forme sèche.

6. Bandelette d'immunochromatographie selon l'une quelconque des revendications 1 à 5, comprenant :
(i) un premier ensemble de particules, colorées d'une première couleur, lesdites particules étant déshydratées ou sous une forme sèche et susceptibles d'être reconstituées par une mise en contact avec l'échantillon de test et/ou une solution tampon, où lesdites premières particules sont liées à, totalement revêtues par, ou partiellement revêtues par l'antigène, ou un fragment de celui-ci, du réactif de détection qui se lie spécifiquement à un anticorps anti-ASFV,
(ii) un deuxième ensemble de particules, colorées d'une deuxième couleur, où ladite deuxième couleur est la même que, ou différente de, ladite première couleur, de préférence ladite deuxième couleur est différente de ladite première couleur, lesdites particules étant déshydratées ou sous une forme sèche et susceptibles d'être reconstituées par une mise en contact avec l'échantillon de test et/ou une solution tampon, où lesdites deuxièmes particules sont liées à, totalement revêtues par, ou partiellement revêtues par l'antigène, ou un fragment de celui-ci, du réactif de détection qui se lie spécifiquement à l'anticorps anti-CSFV, et
(iii) un troisième ensemble de particules, colorées d'une troisième couleur, où ladite troisième couleur est la même que, ou différente de, ladite première et/ou deuxième couleur, de préférence ladite troisième couleur est différente de ladite première couleur et de ladite deuxième couleur, les première et deuxième couleurs étant également différentes l'une de l'autre, lesdites particules étant facultativement déshydratées ou sous une forme sèche et susceptibles d'être reconstituées par une mise en contact avec l'échantillon de test, et/ou une solution tampon, où lesdites troisièmes particules sont liées à, totalement revêtues par, ou partiellement revêtues par un réactif de contrôle du réactif de détection de contrôle.

7. Bandelette d'immunochromatographie selon l'une quelconque des revendications 1 à 6, dans laquelle le réactif de contrôle est sélectionné dans le groupe comprenant la biotine, l'avidine, la streptavidine, la ASB, la ASH, la KLH et l'OVA, et le réactif de capture qi se lie spécifiquement audit réactif de contrôle est sélectionné dans le groupe comprenant un anticorps anti-biotine, l'avidine, la streptavidine, un anticorps anti-ASB, un anticorps anti-ASH, un anticorps anti-KLH, et un anticorps anti-OVA.

8. Kit comprenant au moins une bandelette d'immunochromatographie selon l'une quelconque des revendications 1 à 7.

9. Kit selon la revendication 8, dans lequel la bandelette d'immunochromatographie est dans un format simplex ou dans un format multiplex.

10. Utilisation d'une bandelette d'immunochromatographie selon l'une quelconque des revendications 1 à 7, ou d'un kit selon la revendication 8 ou 9, pour détecter des anticorps anti-ASFV et/ou des anticorps anti-CSFV dans un échantillon, ou pour diagnostiquer une infection provoquée par l'ASFV et/ou par le CSFV, ou pour faire la distinction entre l'ASFV et le CSFV en tant qu'agent causal d'une infection chez un sujet.

11. Utilisation d'une bandelette d'immunochromatographie ou d'un kit selon la revendication 10, dans laquelle la détection des anticorps anti-ASFV et/ou des anticorps anti-CSFV, et/ou le diagnostic de l'infection est réalisé par un dosage immunologique basé sur une chromatographie à flux latéral.

12. Procédé de dosage immunologique pour détecter la présence d'anticorps anti-ASFV et/ou d'anticorps anti-CSFV chez un sujet, où ledit procédé de dosage immunologique est réalisé au moyen d'une bandelette d'immunochromatographie selon l'une quelconque des revendications 1 à 7, ledit procédé comprenant
(i) la mise en contact d'un échantillon dudit sujet avec un tampon d'échantillon de la bandelette d'immunochromatographie,
(ii) une incubation, dans des conditions appropriées pour permettre la formation de complexes dans le tampon de conjugaison de ladite bandelette d'immunochromatographie entre l'anticorps anti-ASFV et un réactif de détection qui se lie spécifiquement audit anticorps anti-ASFV, et/ou de complexes entre l'anti-CSFV et un réactif de détection qui se lie spécifiquement audit anticorps anti-CSFV, et la migration desdits complexes vers le tampon de détection de signal de la bandelette d'immunochromatographie, et
(iii) la détection, dans la zone de détection de signal de ladite bandelette d'immunochromatographie, de la formation de complexes entre l'anticorps anti-ASFV-réactif de détection et un réactif de capture qui se lie spécifiquement audit anticorps anti-ASFV et/ou de complexes entre l'anti-CSFV-réactif de détection et un réactif de capture qui se lie spécifiquement audit anticorps anti-CSFV,
dans lequel la détection desdits complexes entre l'anticorps anti-ASFV et un réactif de capture qui se lie spécifiquement audit anticorps anti-ASFV indique la présence d'anticorps anti-ASFV chez ledit sujet, et/ou la détection desdits complexes entre l'anticorps anti-CSFV et un réactif de capture qui se lie spécifiquement audit anticorps anti-CSFV indique la présence d'anticorps anti-CSFV chez ledit sujet.

13. Procédé de dosage immunologique pour faire la distinction entre l'agent causale d'une infection chez un sujet, où ledit agent causal est sélectionné dans le groupe consistant en l'ASFV, le CSFV et des combinaisons de ceux-ci, où ledit procédé de dosage immunologique est réalisé au moyen d'une bandelette d'immunochromatographie selon l'une quelconque des revendications 1 à 7, ledit procédé comprenant
(i) la mise en contact d'un échantillon dudit sujet avec un tampon d'échantillon de la bandelette d'immunochromatographie,
(ii) une incubation, dans des conditions appropriées pour permettre la formation de complexes dans le tampon de conjugaison de ladite bandelette d'immunochromatographie entre l'anticorps anti-ASFV et un réactif de détection qui se lie spécifiquement audit anticorps anti-ASFV et/ou de complexes entre l'anti-CSFV et un réactif de détection qui se lie spécifiquement audit anticorps anti-CSFV, et la migration desdits complexes vers le tampon de détection de signal de la bandelette d'immunochromatographie, et
(iii) la détection, dans la zone de détection de signal de ladite bandelette d'immunochromatographie, de la formation de complexes entre l'anticorps anti-ASFV-réactif de détection et un réactif de capture qui se lie spécifiquement audit anticorps anti-ASFV et/ou de complexes entre l'anti-CSFV-réactif de détection et un réactif de capture qui se lie spécifiquement audit anticorps anti-CSFV,
dans lequel la détection desdits complexes entre l'anticorps anti-ASFV et un réactif de capture qui se lie spécifiquement audit anticorps anti-ASFV indique la présence d'anticorps anti-ASFV chez ledit sujet, et la présence desdits anticorps anti-ASFV indique que l'agent causal de ladite infection est l'ASFV ; et/ou
dans lequel la détection desdits complexes entre l'anticorps anti-CSFV et un réactif de capture qui se lie spécifiquement audit anticorps anti-CSFV indique la présence d'anticorps anti-CSFV chez ledit sujet, et la présence desdits anticorps anti-CSFV indique que l'agent causal de ladite infection est le CSFV.

14. Procédé de dosage immunologique selon la revendication 12 ou 13, dans lequel l'échantillon est sélectionné dans le groupe comprenant du sang total, du sérum, du plasma, des jus de viande et de la salive.
